(19) Europäisches Patentamt
European Patent Office
Office européen des brevets

(11) **EP 1 932 851 B1**

(12) **EUROPEAN PATENT SPECIFICATION**

(45) Date of publication and mention of the grant of the patent:
**12.06.2013 Bulletin 2013/24**

(21) Application number: **06821802.3**

(22) Date of filing: **08.09.2006**

(51) Int Cl.:
***C07F 15/00*** *(2006.01)* ***C07D 207/44*** *(2006.01)*
***C07D 213/53*** *(2006.01)* ***C09D 11/00*** *(2006.01)*
***C09K 11/06*** *(2006.01)* ***H01L 51/50*** *(2006.01)*

(86) International application number:
**PCT/JP2006/317895**

(87) International publication number:
**WO 2007/046201 (26.04.2007 Gazette 2007/17)**

(54) **METAL COMPLEX, LIGHT-EMITTING MATERIAL, AND LIGHT-EMITTING DEVICE**

METALLKOMPLEX, LICHTEMITTIERENDES MATERIAL UND LICHTEMITTIERENDES GERÄT

COMPLEXE METALLIQUE, MATERIAU ELECTROLUMINESCENT, ET DISPOSITIF ELECTROLUMINESCENT

(84) Designated Contracting States:
**DE GB**

(30) Priority: **09.09.2005 JP 2005261841**

(43) Date of publication of application:
**18.06.2008 Bulletin 2008/25**

(73) Proprietor: **Sumitomo Chemical Company, Limited**
**Tokyo 104-8260 (JP)**

(72) Inventors:
- **AKINO, Nobuhiko**
**Koshigaya-shi, Saitama, 343-0807 (JP)**
- **SEKINE, Chizu**
**Tsukuba-shi, Ibaraki, 300-4249 (JP)**
- **OKAMURA, Rei**
**Tsukuba-shi, Ibaraki, 305-0821 (JP)**

(74) Representative: **Benson, John Everett et al**
**J A Kemp**
**14 South Square**
**Gray's Inn**
**London WC1R 5JJ (GB)**

(56) References cited:
**JP-A- 2003 261 568** **JP-A- 2004 296 170**
**US-A1- 20040 183 082**

- **MIYAZAKI Y. ET AL.: 'Preparation of new type of azacalixarene, azacalix[n](2,6)pyridine' TETRAHEDRON LETTERS vol. 43, no. 44, 2002, pages 7945 - 7948, XP004385586**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 932 851 B1

## Description

TECHNICAL FIELD

The present invention relates to a metal complex, a light-emitting material containing the metal complex and a light-emitting device.

BACKGROUND ART

As a light-emitting material used in a light-emitting layer of an electroluminescent device, a metal complex emitting light from a triplet excited state or a light-emitting material containing the metal complex can be expected to provide a higher luminous efficiency than a fluorescent material, which is frequently used therein, emitting light from a singlet excited state. This is because 25% of the excitons produced by recombination of carriers are theoretically in the singlet excited state, whereas the remaining 75% of the excitons are in the triplet excited state. In other words, when light emission (fluorescence) from the singlet excited state is used, theoretically, 25% is the upper limit. In contrast, when light emission (phosphorescence) from the triplet excited state is used, theoretically, 3-fold luminous efficiency can be expected. In addition, if the intersystem crossing occurs efficiently from the singlet excited state (25%) to the triplet excited state, theoretically 4 fold luminous efficiency can be expected based on the relative energy relationship.

Generally, the light-emission (phosphorescence) from the triplet excited state, which involves the transition from the triplet excited state to the singlet ground state, is inevitably accompanied by spin inversion. Therefore, this is forbidden transition. However, some metal complex compounds containing a heavy metal have been found to emit light since the forbidden transition is overcome by the heavy-atom effect. As an example of the metal complex compounds emitting light from the triplet excited state, mention may be made of Tris-Ortho-Metalated Complex of Iridium(III) with 2-Phenylpyridine ($Ir(ppy)_3$), which contains iridium as a central metal and is known to emit green light with high efficiency. Also, an electroluminescent device has been reported, which is formed by laminating the metal complex in combination with a low-molecular weight host material (non-patent document 1).

Non-Patent Document 1: APPLIED PHYSICS LETTERS, Vol. 75, No. 1, p. 4 (1999)
US-A-2004/0183082 describes a high-efficient white emission light emitting element.

DISCLOSURE OF THE INVENTION

Problem to be solved by the Invention

However, iridium is a metal limited in production and reserve. Therefore, it is difficult to produce a light-emitting device using iridium in a large amount and at low cost. For this reason, it has been desired to use a metal having a larger production and reserve and obtained at a lower cost than iridium to develop a phosphorescent metal complex having excellent luminous efficiency, stability and color purity, etc.

The object of the present invention is therefore to provide a novel light-emitting metal complex using a metal having a larger production and reserve than iridium as a central metal and excellent in luminous efficiency, stability and color purity, etc., and to provide a photoelectric device using the novel light-emitting metal complex.

Means for solving the Problem

More specifically, according to the present invention, there is provided a metal complex characterized by having a partial structure represented by the general formula (1-1) below:

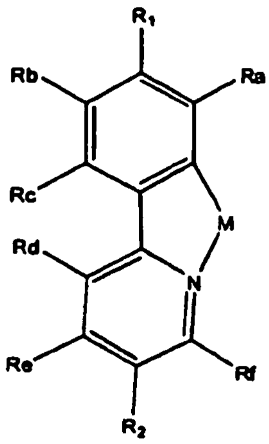

(1-1)

wherein M represents the central metal; $R_1$ and $R_2$ and Ra to Rf each independently represent a hydrogen atom, an alkyl group, an alkoxy group, a phenyl group or a halogen atom, provided that at least one of $R_1$ and $R_2$ is a group represented by the formula (2) below,
wherein $R_{11}$ to $R_{19}$ each independently represent a hydrogen atom, an alkyl group, an alkoxy group, a phenyl group or a halogen atom, and the wave line indicates a binding position.

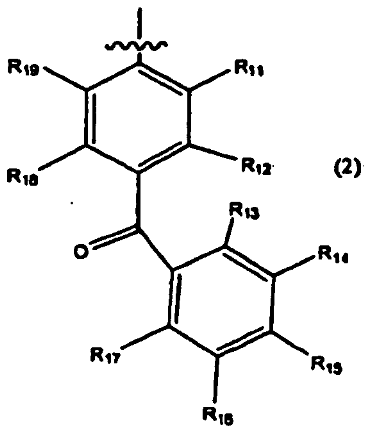

(2)

Typically, the metal complex has a central metal M selected from the group consisting of Sc, Ti, V, Cr, Mn, Fe, Co, Ni, Cu, Zn, Y, Zr, Nb, Mo, Tc, Ru, Rh, Pd, Ag, Cd, Hf, Ta, W, Re, Pt, Au and Hg, wherein no halogen unidentate ligand is contained in the complex; and a ratio of a sum of squares of orbital coefficients of outermost d orbitals of the central metal relative to a sum of squares of all atomic orbital coefficients in a highest occupied molecular orbital (HOMO) obtained by a computational scientific technique is 1/3 or more; an energy difference (S1-T1) between a lowest singlet excitation energy (S1) and a lowest triplet excitation energy (T1) obtained by a computational scientific technique falls within 0.1 (eV) to 1.0 (eV) (both inclusive); and an oscillator strength (f) in the lowest singlet excited state falls within a range of 0.005 to 1.0 (both inclusive).

Advantages of the Invention

A metal complex according to the present invention is a complex using a metal having a larger production and reserve than iridium as a central metal and excellent in luminous efficiency, stability and color purity, etc. Therefore, use of the metal complex of the present invention in a photoelectric device such as electroluminescent device can render the characteristics of the device to be more excellent.

BRIEF DESCRIPTION OF THE DRAWINGS

Fig. 1 is a graph showing correlation between quantum efficiency and the ratio of d orbital (%) with respect to Examples 1 to 3 according to the present invention and Comparative Example 1. The relative quantum efficiency indicates a relative value based on the quantum efficiency of Comparative Example 1 as 1.

BEST MODE FOR CARRYING OUT THE INVENTION

The present invention will now be described more specifically below.
The present inventors intensively conducted studies. As a result, they found that, the ratio of the sum of the squares of the orbital coefficients of the outermost d orbitals of the central metal relative to the sum of the squares of all atomic orbital coefficients in the highest occupied molecular orbital (HOMO) obtained by a computational scientific technique has strong correlation with luminous efficiency. Based on the finding, the present invention has been attained. To describe more specifically, in a metal complex characterized in that the ratio is 1/3 or more, excellent properties in luminous efficiency and stability can be obtained. The ratio of the sum of the squares of the orbital coefficients of the outermost d orbitals of the central metal relative to the sum of the squares of all atomic orbital coefficients in the highest occupied molecular orbital (HOMO) obtained by a computational scientific technique is preferably 1/3 or more as described above, more specifically, 1/3 to 2/3 (both inclusive), more preferably, 7/20 to 2/3 (both inclusive), further preferably, 2/5 to 2/3 (both inclusive), and particularly preferably, 1/2 to 2/3 (both inclusive).

The central metal may be one of the metal selected from the group consisting of Sc, Ti, V, Cr, Mn, Fe, Co, Ni, Cu, Zn, Y, Zr, Nb, Mo, Tc, Ru, Rh, Pd, Ag, Cd, Hf, Ta, W, Re, Pt, Au and Hg. To obtain high luminous efficiency, Y, Zr, Nb, Mo, Tc, Ru, Rh, Pd, Ag, W, Re, Pt and Au are preferable; Ru, Rh, Pd, W, Re, Pt and Au are more preferable; Re and Pt are further preferable; and Pt is the most preferable.

As the computational scientific technique to be used for calculating the aforementioned ratio, there are known methods based on a semi-empirical technique and a non-empirical technique, such as a molecular orbital method and a density functional method. For example, to obtain an optimized structure and excitation energy, Hartree-Fock (HF) method or a density functional method may be used. In the present invention, using a quantum chemical calculation program, Gaussian03 and based on the density functional method of the B3LYP level, the structure of a metal complex in the basic state was optimized and population analysis of each orbital was performed. In this way, the ratio occupied by the outermost d orbital of a central metal in the HOMO was obtained. At this time, as the basis function, LANL2DZ was applied to the central metal, whereas 6-31G* to other atoms besides the central metal. The population analysis in a metal complex was
performed as follows. More specifically, the ratio $\rho_d^{HOMO}$(%) of the sum of the squares of the orbital coefficients of the outermost d orbitals of the central metal relative to the sum of the squares of all atomic orbital coefficients in the HOMO of the metal complex was calculated in accordance with the following equation:

$$\rho_d^{HOMO}(\%) = \Sigma_{id}(C_{id}^{HOMO})^2/\Sigma_n(C_n^{HOMO})^2 \times 100 \quad (\%)$$

In the formula, id and n represent the number of d orbitals and the number of all atomic orbitals, which are used in the computational technique and basis function. $C_{id}^{HOMO}$ and $C_n^{HOMO}$ each represent the coefficients of the atomic orbitals represented by id and n in the HOMO. The population analysis for a metal complex is, for example, described in JOURNAL OF PHYSICAL CHEMISTRY A 2002, Vol. 106, p. 1634. Furthermore, the lowest singlet excitation energy, the lowest triplet excitation energy and oscillator strength were obtained by a time-dependent density functional method of the B3LYP level using the same basis as above, after optimization of a structure.

The reason why the light emitted from an electroluminescent device using light emission of a metal complex deteriorates has not yet been elucidated.

Generally, light-emission (phosphorescence) from the triplet excited state involving the transition from the triplet excited state to the singlet ground state is forbidden transition. Therefore, the triplet excited state lasts longer than a general singlet excited state. In general, the triplet excited state lasts longer by several digits or more. As a result, the unstable excitation state having high energy lasts longer. Accordingly, an inactivation process through reactions with adjacent compounds frequently occurs. In addition, a large number of metal complexes in the triplet excited state are present in a saturated condition, likely causing a phenomenon known as so-called triplet-triplet annihilation. These phenomena conceivably affect a phosphorescent efficiency.

More specifically, the present inventors found that stable and high efficient light emission is obtained preferably by using a metal complex whose forbidden transition is easily overcome and whose lifetime of triplet excited state is short, and that luminous efficiency can be expressed by the ratio of the outermost orbital d of a central metal in the HOMO, as described above. Furthermore, the present inventors found it preferable in view of the efficiency that the unoccupied $\pi$ * orbital of a ligand mostly contributes to the lowest unoccupied molecular orbital (LUMO).

Furthermore, to use phosphorescence to a maximum, the oscillator strength (f) in the lowest singlet excited state must take a numerical value except 0. Generally, the lower limit of the oscillator strength (f) is preferably at least 0.005 or more, more preferably, 0.01 or more, further preferably, 0.025 or more, and particularly preferably, 0.035 or more. In consideration of general properties of a metal complex, the upper limit of the oscillator strength (f) is 1.0 or less,

preferably 0.5 or less, more preferably, 0.3 or less and particularly preferably, 0.25 or less. Furthermore, it is preferable that the transition (intersystem crossing) from the singlet excited state to the triplet excited state is likely to occur. From this, the energy difference (S1-T1) between the lowest triplet excitation energy (T1) is preferably low. Generally, the upper limit of the difference is preferably 1.0 (eV) or less and preferably 0.8 (eV) or less. The lower limit of the energy difference (S1-T1) is generally 0.1 (eV) or more, preferably 0.12 (eV) or more, and further preferably, 0.15 (eV) or more. The oscillator strength (f) is preferably balanced with the energy difference (S1-T1), appropriately. Generally, they preferably fall within the range defined by the equations:

$$f \leq 0.24 \times (S1-T1) + 0.06$$

$$f \geq 0.24 \times (S1-T1) - 0.06$$

more preferably, falls within the range defined by the equations:

$$f \leq 0.24 \times (S1-T1) + 0.05$$

$$f \geq 0.24 \times (S1-T1) - 0.05$$

further preferably, falls within the range defined by the equations:

$$f \leq 0.24 \times (S1-T1) + 0.04$$

$$f \geq 0.24 \times (S1-T1) - 0.04$$

and particularly preferably, falls within the range defined by the equations:

$$f \leq 0.24 \times (S1-T1) + 0.03$$

$$f \geq 0.24 \times (S1-T1) - 0.03.$$

A further preferable state of balance slightly differs depending upon the range of the energy difference (S1-T1). When the value of the energy difference (S1-T1) is larger than 0.28 (eV), the oscillator strength (f) is preferably 0.035 or more, more preferably, 0.07 to 0.3 (both inclusive), further preferably, 0.09 to 0.3 (both inclusive) and particularly preferably, 0.1 to 0.25 (both inclusive) in addition to the aforementioned conditions. In this case, the energy difference (S1-T1) is preferably 0.8 (eV) or less, further preferably, 0.6 (eV) or less, and particularly preferably, 0.5 (eV) or less. On the other hand, when the energy difference (S1-T1) falls within the range of 0.1 (eV) to 0.28 (eV), the oscillator strength (f) is preferably 0.015 or more, further preferably, 0.025 or more, and more preferably, 0.025 to 0.05 (both inclusive) in addition to the aforementioned conditions. In this case, the energy difference (S1-T1) preferably falls within the range of 0.12 (eV) to 0.28 (eV)(both inclusive), more preferably, 0.15 (eV) to 0.28 (eV)(both inclusive) and further preferably, 0.15 (eV) to 0.26 (eV)(both inclusive).

Metal complexes include ionic complexes and nonionic complexes. When an ionic metal complex is used, for example, in an organic EL device, a counter ion may be required in some cases to neutralize the entire system. However, when the counter ion interacts with the metal complex, stability may decrease, the light-emission wavelength may change, and color purity may decrease. Since these problems are likely to occur, a metal complex is preferably nonionic (neutral).

Next, a ligand of a metal complex according to the present invention will be described.

The ligand affects color and intensity of light emitted from a metal complex. For example, in the case of (1,10)-phenanthroline)$Re(CO)_3(C1)$ complex, which is a metal complex containing a number of unidentate ligands such as a carbonyl group (CO) and a halogen group (Cl), there are an energy inactivation process due to unidentate ligands and a problem with stability. As a result, the luminous efficiency and stability may decrease. Furthermore, in view of luminous efficiency

and stability, a central metal is preferably blocked by ligands. For this reason, no halogen unidentate ligands, which cannot have a substituent, are preferably included.

To prevent decreases of the luminous efficiency and stability, the number of unidentate ligands is preferably reduced. A metal complex preferably has at least one multidentate chelate ligand having at least one aromatic ring. The multidentate chelate ligand used herein may be a bidentate, tridentate or tetradentate ligand.

In the case where a metal complex according to the present invention has a unidentate ligand, examples of the unidentate ligand may include an alkynyl group, aryloxy group, amino group, silyl group, acyl group, alkenyl group, alkyl group, alkoxy group, alkylthio group, arylthio group, enolate group, amide group, alkyl group, aryl group, heterocyclic ligand, carboxyl group, amide group, imido group, alkoxy group, alkylmercapto group, carbonyl ligand, alkene ligand, alkyne ligand, amine ligand, imine ligand, nitrile ligand, isonitrile ligand, phosphine ligand, phosphine oxide ligand, phosphite ligand, ether ligand, sulfone ligand, sulfoxide ligand and sulfide ligand. Each ligand may be substituted with a halogen atom such as fluorine or chlorine.

The unidentate ligand preferably has an aromatic ring. Further preferably, the ligand atom in the aromatic ring is carbon or nitrogen; or the aromatic ring is a condensed ring.

Examples of the alkyl group include a methyl group, ethyl group, propyl group, butyl group and cyclohexyl group. Examples of the aryl group include a phenyl group, tolyl group, 1-naphthyl group and 2-naphthyl group. The heterocyclic ligand may be nonvalent or monovalent. Examples of the nonvalent heterocyclic ligand include 2,2'-bipyridyl, 1,10-phenanthroline, 2-(4-thiophen-2-yl)pyridine and 2-(benzothiophen-2-yl)pyridine. Examples of the monovalent heterocyclic ligand include phenylpyridine, 2-(paraphenylphenyl)pyridine, 7-bromobenzo[h]quinoline, 2-(4-phenylthiophen-2-yl)pyridine, 2-phenylbenzoxazole, 2-(paraphenylphenyl)benzoxazole, 2-phenylbenzothiazole and 2-(paraphenylphenyl)benzothiazole.

Examples of the carboxyl group include, but not particularly limited to, an acetoxy group, naphthenate group and 2-ethylhexanoate group. Examples of the amide group include, but not particularly limited to, a dimethylamide group, diethylamide group, diisopropylamide group, dioctylamide group, didecylamide group, didodecylamide group, bis(trimethylsilyl)amide group, diphenylamide group, N-methylanilide and anilide group. Examples of the imido group include, but not particularly limited to, benzophenone imido. Examples of the alkoxyl group include, but not particularly limited to, a methoxy group, ethoxy group, propoxy group, butoxy group and phenoxy group. Examples of the alkylmercapto group include, but not particularly limited to, methylmercapto group, ethylmercapto group, propylmercapto group, butylmercapto group and phenylmercapto group. Examples of the carbonyl ligand include carbon monoxide, ketones such as acetone and benzophenone; diketones such as acetylacetone and acenaphthoquinone; and acetonate ligands such as acetylacetonato, dibenzomethylate and thenoyltrifluoroacetonato. Examples of the alkene ligand include, but not particularly limited to, ethylene, propylene, butene, hexene and decene. Examples of the alkyne ligand include, but not particularly limited to, acetylene, phenylacetylene and diphenylacetylene. Examples of the amine ligand include, but not particularly limited to, triethylamine and tributylamine. Examples of the imine ligand include, but not particularly limited to, benzophenone imine and methylethylketone imine. Examples of the nitrile ligand include, but not particularly limited to, acetonitrile and benzonitrile. Examples of the isonitrile ligand include, but not particularly limited to, t-butylisonitrile and phenylisonitirile. Examples of the phosphine ligand include, but not particularly limited to, triphenylphosphine, tritolylphosphine and tricyclohexylphosphine and tributylphosphine. Examples of the phosphine oxide ligand include, but not particularly limited to, tributylphosphine oxide and triphenylphosphine oxide. Examples of the phosphite ligand include, but not particularly limited to, triphenylphosphite, tritolylphosphite, tributylphosphite and triethyl phosphite. Examples of the ether ligand include, but not particularly limited to, dimethyl ether, diethyl ether and tetrahydrofuran. Examples of the sulfone ligand include, but not particularly limited to, dimethylsulfone and dibutylsulfone. Examples of the sulfoxide ligand include, but not particularly limited to, dimethylsulfoxide and dibutylsulfoxide. Examples of the sulfide ligand include, but not particularly limited to, ethylsulfide and butylsulfide.

A metal complex according to the present invention preferably contains at least one multidentate chelate ligand containing at least one aromatic ring. Examples of the aromatic ring include aromatic hydrocarbon rings such as a benzene ring and naphthalene ring and aromatic heterocyclic rings such as pyridine.

When a metal complex according to the present invention contains a multidentate chelate ligand, the multidentate chelate ligand preferably contains an aromatic ring. The aromatic ring may have a substituent. Examples of the substituent may include a halogen atom, alkyl group, alkyloxy group, alkylthio group, aryl group, aryloxy group, arylthio group, arylalkyl group, arylalkyloxy group, arylalkylthio group, acyl group, acyloxy group, amide group, acid imido group, imine residue, substituted amino group, substituted silyl group, substituted silyloxy group, substituted silylthio group, substituted silylamino group, monovalent heterocyclic group, heteroaryloxy group, heteroarylthio group, arylalkenyl group, arylethynyl group, substituted carboxyl group and cyano group. When a plurality of substituents are present on an aromatic ring, they may be the same or different.

Examples of the halogen atom include a fluorine atom, chlorine atom, bromine atom and iodine atom.

As the alkyl group, straight, branched or cyclic alkyl group may be used. The number of carbon atoms is generally about 1 to 10 and preferably 3 to 10. Specific examples thereof may include a methyl group, ethyl group, propyl

group, isopropyl group, butyl group, isobutyl group, t-butyl group, pentyl group, hexyl group, cyclohexyl group, heptyl group, octyl group, 2-ethylhexyl group, nonyl group, decyl group, 3,7-dimethyloctyl group, lauryl group, trifluoromethyl group, pentafluoroethyl group, perfluorobutyl group, perfluorohexyl group and perfluorooctyl group. Of them, a pentyl group, hexyl group, octyl group, 2-ethylhexyl group, decyl group and 3,7-dimethyloctyl group are preferable.

As the alkyloxy group, straight, branched or cyclic may be used. The number of carbon atoms is generally about 1 to 10 and preferably 3 to 10. Specific examples thereof may include a methoxy group, ethoxy group, propyloxy group, isopropyloxy group, butoxy group, isobutoxy group, t-butoxy group, pentyloxy group, hexyloxy group, cyclohexyloxy group, heptyloxy group, octyloxy group, 2-ethylhexyloxy groups, nonyloxy group, decyloxy group, 3,7-dimethyl octyloxy group, lauryloxy group, trifluoromethoxy group, pentafluoroethoxy group, perfluorobutoxy group, perfluorohexyl group, perfluorooctyl group, methoxymethyloxy group, 2-methoxyethyloxy group. Of them, a pentyloxy group, hexyloxy group, octyloxy group, 2-ethylhexyloxy group, decyloxy group and 3,7-dimethyloctyloxy group are preferable.

As the alkylthio group, straight, branched or cyclic may be used. The number of carbon atoms is generally about 1 to 10 and preferably 3 to 10. Specific examples thereof may include a methylthio group, ethylthio group, propylthio group, isopropylthio group, butylthio group, isobutylthio group, t-butylthio group, pentylthio group, hexylthio group, cyclohexylthio group, heptylthio group, octylthio group, 2-ethylhexylthio group, nonylthio group, decylthio group, 3,7-dimethyloctylthio groups, laurylthio group and trifluoromethylthio group. Of them, a pentylthio group, hexylthio group, octylthio group, 2-ethylhexylthio group, decylthio group and 3,7-dimethyloctylthio group are preferable.

The aryl group generally has 6 to 60 carbon atoms and preferably 7 to 48 carbon atoms. Specific examples thereof may include a phenyl group, $C_1$-$C_{12}$ alkoxyphenyl group ($C_1$-$C_{12}$ represents 1 to 12 carbon atoms. The same definition will be used hereinafter), $C_1$-$C_{12}$ alkylphenyl group, 1-naphthyl group, 2-naphthyl group, 1-anthracenyl group, 2-anthracenyl group, 9-anthracenyl group and pentafluorophenyl group. Of them, a $C_1$-$C_{12}$ alkoxyphenyl group and $C_1$-$C_{12}$ alkylphenyl group are preferable. The aryl group used herein is an atomic group obtained by subtracting a hydrogen atom from an aromatic hydrocarbon. The aromatic hydrocarbon used herein include an aromatic hydrocarbon having a condensed ring, an aromatic hydrocarbon having at least two independent benzene rings or condensed rings directly bonded or bonded via a group such as vinylene.

Specific examples of the $C_1$-$C_{12}$ alkoxy include methoxy, ethoxy, propyloxy, isopropyloxy, butoxy, isobutoxy, t-butoxy, pentyloxy, hexyloxy, cyclohexyloxy, heptyloxy, octyloxy, 2-ethylhexyloxy, nonyloxy, decyloxy, 3,7-dimethyloctyloxy and lauryloxy.

Specific examples of the $C_1$-$C_{12}$ alkylphenyl group include a methylphenyl group, ethylphenyl group, dimethylphenyl group, propylphenyl group, mesityl group, methylethylphenyl group, isopropylphenyl group, butylphenyl group, isobutylphenyl group, t-butylphenyl group, pentylphenyl group, isoamylphenyl group, hexylphenyl group, heptylphenyl group, octylphenyl group, nonylphenyl group, decylphenyl group and dodecylphenyl group.

The aryloxy group generally has about 6 to 60 carbon atoms and preferably 7 to 48 carbon atoms. Specific examples thereof include a phenoxy group, $C_1$-$C_{12}$ alkoxyphenoxy group, $C_1$-$C_{12}$ alkylphenoxy group, 1-naphthyloxy group, 2-naphthyloxy group and pentafluorophenyloxy group. Of them, a $C_1$-$C_{12}$ alkoxyphenoxy group and $C_1$-$C_{12}$ alkylphenoxy group are preferable.

Examples of $C_1$-$C_{12}$ alkoxy include methoxy, ethoxy, propyloxy, isopropyloxy, butoxy, isobutoxy, t-butoxy, pentyloxy, hexyloxy, cyclohexyloxy, heptyloxy, octyloxy, 2-ethylhexyloxy, nonyloxy, decyloxy, 3,7-dimethyloctyloxy and lauryloxy.

Specific examples of the $C_1$-$C_{12}$ alkylphenoxy group include a methylphenoxy group, ethylphenoxy group, dimethylphenoxy group, propylphenoxy group, 1,3,5-trimethylphenoxy group, methylethylphenoxy group, isopropylphenoxy group, butylphenoxy group, isobutylphenoxy group, t-butylphenoxy group, pentylphenoxy group, isoamylphenoxy group, hexylphenoxy group, heptylphenoxy group, octylphenoxy group, nonylphenoxy group, decylphenoxy group and dodecylphenoxy group.

The arylthio group generally has about 6 to 60 carbon atoms and preferably 7 to 48 carbon atoms. Specific examples thereof include a phenylthio group, $C_1$-$C_{12}$ alkoxyphenylthio group and $C_1$-$C_{12}$ alkylphenylthio group , 1-naphthylthiogroup, 2-naphthylthio group and pentafluorophenylthio group. Of them, a $C_1$-$C_{12}$ alkoxyphenylthio group and $C_1$-$C_{12}$ alkylphenylthio group are preferable.

The arylalkyl group generally has about 7 to 60 carbon atoms and preferably 7 to 48 carbon atoms. Specific examples thereof include a phenyl-$C_1$-$C_{12}$ alkyl group, $C_1$-$C_{12}$ alkoxyphenyl-$C_1$-$C_{12}$ alkyl group, $C_1$-$C_{12}$ alkylphenyl-$C_1$-$C_{12}$ alkyl group, 1-naphthyl-$C_1$-$C_{12}$ alkyl group and 2-naphthy-$C_1$-$C_{12}$ alkyl group. Of them, a $C_1$-$C_{12}$ alkoxyphenyl-$C_1$-$C_{12}$ alkyl group and $C_1$-$C_{12}$ alkylphenyl-$C_1$-$C_{12}$ alkyl group are preferable.

The arylalkyloxy group generally has about 7 to 60 carbon atoms and preferably 7 to 48 carbon atoms. Specific examples thereof include phenyl-$C_1$-$C_{12}$ alkoxy groups such as a phenylmethoxy group, phenylethoxy group, phenylbutoxy group, phenylpentyloxy group, phenylhexyloxy group phenylheptyloxy group and phenyloctyloxy group, $C_1$-$C_{12}$ alkoxyphenyl-$C_1$-$C_{12}$ alkoxy group, $C_1$-$C_{12}$ alkylphenyl-$C_1$-$C_{12}$ alkoxy group, 1-naphthyl-$C_1$-$C_{12}$ alkoxy group and 2-naphthyl-$C_1$-$C_{12}$ alkoxy group. Of them, $C_1$-$C_{12}$ alkoxyphenyl-$C_1$-$C_{12}$ alkoxy group and $C_1$-$C_{12}$ alxylphenyl-$C_1$-$C_{12}$ alkoxy group are preferable.

The arylalkylthio group generally has about 7 to 60 carbon atoms and preferably 7 to 48 carbon atoms. Specific

examples thereof include phenyl-$C_1$-$C_{12}$ alkylthio group, $C_1$-$C_{12}$-alkoxyphenyl-$C_1$-$C_{12}$ alkylthio group, $C_1$-$C_{12}$ alxylphenyl-$C_1$-$C_{12}$ alkylthio group, 1-naphthyl-$C_1$-$C_{12}$ alkylthio group and 2-naphthyl-$C_1$-$C_{12}$ alkylthio group. Of them, $C_1$-$C_{12}$ alkoxyphenyl-$C_1$-$C_{12}$ alkylthio group and $C_1$-$C_{12}$ alxylphenyl-$C_1$-$C_{12}$ alkylthio group are preferable.

The acyl group generally has about 2 to 20 carbon atoms and preferably 2 to 18 carbon atoms. Specific examples thereof include an acetyl group, propionyl group, butyryl group, isobutyryl group, pivaloyl group, benzoyl group, trifluoroacetyl group and pentafluorobenzoyl group.

The acyloxy group generally has about 2 to 20 carbon atoms and preferably 2 to 18 carbon atoms. Specific examples thereof include an acetoxy group, propionyloxy group, butyryloxy group, isobutyryloxy group, pivaloyloxy group, benzoyloxy group, trifluoroacetyloxy group and pentafluorobenzoyloxy group.

The amide group generally has about 2 to 20 carbon atoms and preferably 2 to 18 carbon atoms. Specific examples thereof include a formamide group, acetamido group, propioamide group, butyroamide group, benzamide group, trifluoroacetamido group, pentafluorobenzamide group, diformamide group, diacetamide group, dipropioamide group, dibutyroamide group, dibenzamide group, ditrifluoroacetamido group and dipentafluorobenzamide group.

The acid imido group is a residue obtained by removing a hydrogen atom bonded to a nitrogen atom from an acid imido. The number of carbon atoms thereof is generally about 2 to 60 and preferably 2 to 48.
Specific examples thereof include the groups shown below:

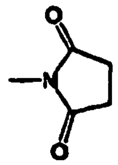
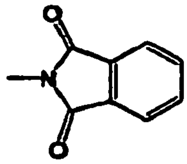
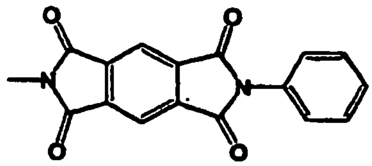
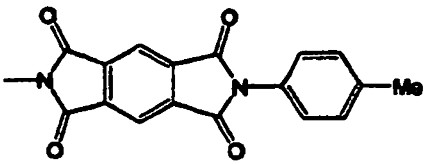
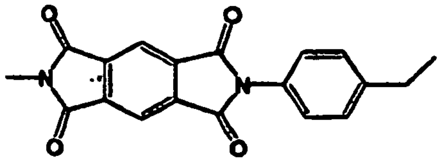
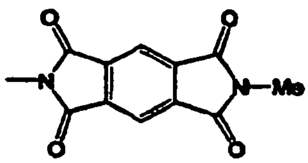
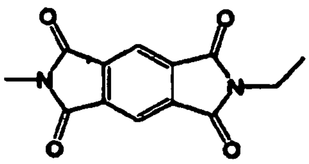
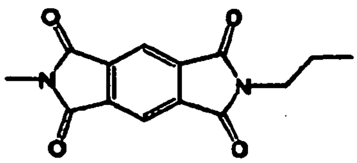
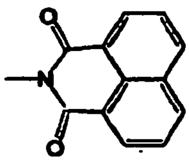
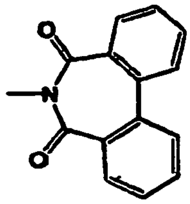

The imine residue is a residue obtained by removing a single hydrogen atom from an imine compound (which refers to an organic compound having -N=C- in the molecule. Examples thereof include aldimine, ketimine and compounds whose hydrogen atom present on N is substituted with an alkyl group or the like). The number of carbon atoms thereof is generally about 2 to 20 and preferably 2 to 18. Specific examples thereof include the groups represented by the following structural formulas:

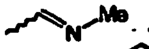 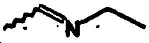 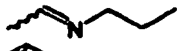 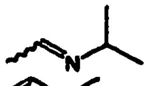 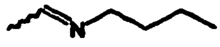 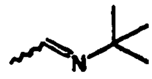

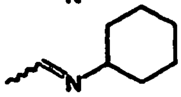 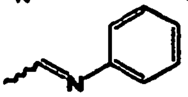 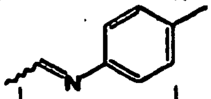

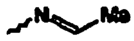 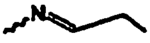 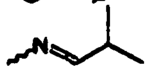 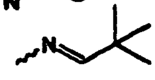 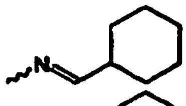 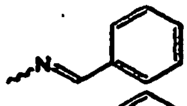

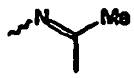 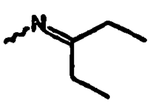 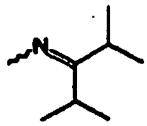 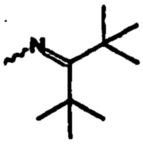 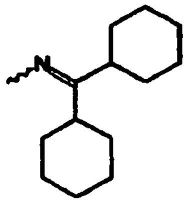 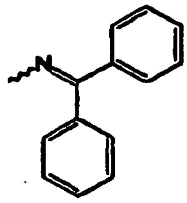

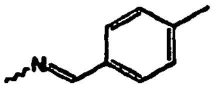 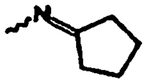 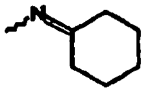

The substituted amino group refers to an amino group substituted with one or two groups selected from an alkyl group, aryl group, arylalkyl group and monovalent heterocyclic group. The alkyl group, aryl group, arylalkyl group and monovalent heterocyclic group may have a substituent. The number of carbon atoms thereof excluding the number of those of the substituent is generally about 1 to 60 and preferably 2 to 48.

Specific examples thereof include a methylamino group, dimethylamino group, ethylamino group, diethylamino group, propylamino group, dipropylamino group, isopropylamino group, diisopropylamino group, butylamino group, isobutylamino group, t-butylamino group, pentylamino group, hexylamino group, cyclohexylamino group, heptylamino group, octylamino group, 2-ethylhexylamino group, nonylamino group, decylamino group, 3,7-dimethyloctylamino group, laurylamino group, cyclopentylamino group, dicyclopentylamino group, cyclohexylamino group, dicyclohexylamino group, pyrrolidyl group, piperidyl group, ditrifluoromethylamino group, phenylamino group, diphenylamino group, $C_1$-$C_{12}$ alkyloxyphenylamino group, di($C_1$-$C_{12}$ alkoxyphenyl) amino group, di($C_1$-$C_{12}$ alkylphenyl)amino group, 1-naphthylamino group, 2-naphthylamino group, pentafluorophenylamino group, pyridylamino group, pyridazinyl amino group, pyrimidylamino group, pyrazylamino group, triazylamino group, phenyl-$C_1$-$C_{12}$ alkylamino group, $C_1$-$C_{12}$ alkoxyphenyl-$C_1$-$C_{12}$ alkylamino group, $C_1$-$C_{12}$ alkylphenyl-$C_1$-$C_{12}$ alkylamino group, di ($C_1$-$C_{12}$ alkoxyphenyl-$C_1$-$C_{12}$ alkyl)amino group, di ($C_1$-$C_{12}$ alkylphenyl-$C_1$-$C_{12}$ alkyl) amino group, 1-naphthyl-$C_1$-$C_{12}$ alkylamino group and 2-naphthyl-$C_1$-$C_{12}$ alkylamino group.

The substituted silyl group refers to a silyl group substituted with 1, 2 or 3 groups selected from an alkyl group, aryl group, arylalkyl group and monovalent heterocyclic group. The number of carbon atoms thereof is generally about 1 to 60 and preferably 3 to 48. Note that the alkyl group, aryl group, arylalkyl group and monovalent heterocyclic group may have a substituent.

Specific examples thereof include a trimethylsilyl group, triethylsilyl group, tripropylsilyl group, triisopropylsilyl group, dimethylisopropylsilyl group, diethylisopropylsilyl group, t-butylsilyldimethylsilyl group, pentyldimethylsilyl group, hexyldimethylsilyl group, heptyldimethylsilyl group, octyldimethylsilyl group, 2-ethylhexyl-dimethylsilyl group, nonyldimethylsilyl group, decyldimethylsilyl group, 3,7-dimethyloctyl-dimethylsilyl group, lauryldimethylsilyl group, phenyl-$C_1$-$C_{12}$ alkylsilyl group, $C_1$-$C_{12}$ alkoxyphenyl-$C_1$-$C_{12}$ alkylsilyl group, $C_1$-$C_{12}$ alkylpheny-$C_1$-$C_{12}$ alkylsilyl group, 1-naphthyl-$C_1$-$C_{12}$ alkylsilyl group, 2-naphthyl-$C_1$-$C_{12}$ alkylsilyl group, phenyl-$C_1$-$C_{12}$ alkyldimethylsilyl group, triphenylsilyl group, tri-p-xylylsilyl group, tribenzylsilyl group, diphenylmethylsilyl group, t-butyldiphenylsilyl group and dimethylphenylsilyl group.

The substituted silyloxy group refers to a silyloxy group substituted with 1, 2 or 3 groups selected from an alkyloxy group, aryloxy group, arylalkyloxy group and monovalent heterocyclic oxy group. The number of carbon atoms thereof is generally about 1 to 60 and preferably 3 to 48. Note that the alkyloxy group, aryloxy group, arylalkyloxy group and monovalent heterocyclic oxy group may have a substituent.

Specific examples thereof include a trimethylsilyloxy group, triethylsilyloxy group, tripropylsilyloxy group, triisopropylsilyloxy group, dimethylisopropylsilyloxy group, diethylisopropylsilyloxy group, t-butylsilyldimethylsilyl group, pentyldimethylsilyloxy group, hexyldimethylsilyloxy group, heptyldimethylsilyloxy group, octyldimethylsilyloxy group, 2-ethylhexyldimethylsilyloxy group, nonyldimethylsilyloxy group, decyldimethylsilyloxy group, 3,7-dimethyloctyl-dimethylsilyloxy group, lauryldimethylsilyloxy group, phenyl-$C_1$-$C_{12}$ alkylsilyloxy group, $C_1$-$C_{12}$ alkoxyphenyl-$C_1$-$C_{12}$ alkylsilyloxy group, $C_1$-$C_{12}$ alkylpheny-$C_1$-$C_{12}$ alkylsilyloxy group, 1-naphthyl-$C_1$-$C_{12}$ alkylsilyloxy group, 2-naphthyl-$C_1$-$C_{12}$ alkylsilyloxy group, phenyl-$C_1$-$C_{12}$ alkyldimethylsilyloxy group, triphenylsilyloxy group, tri-p-xylylsilyloxy group, tribenzylsilyloxy group, diphenylmethylsilyloxy group, t-butyldiphenylsilyloxy group and dimethylphenylsilyloxy group.

The substituted silylthio group refers to a silylthio group substituted with 1, 2 or 3 groups selected from an alkylthio group, arylthio group, arylalkylthio group and monovalent heterocyclic thio group. The number of carbon atoms thereof is generally about 1 to 60 and preferably 3 to 48. Note that the alkyloxy group, arylthio group, arylalkylthio group and monovalent heterocyclic thio group may have a substituent.

Specific examples thereof include a trimethylsilylthio group, triethylsilylthio group, tripropylsilylthio group, triisopropylsilylthio group, dimethylisopropylsilylthio group, diethylisopropylsilylthio group, t-butylsilyldimethylsilylthio group, pentyldimethylsilylthio group, hexyldimethylsilylthio group, heptyldimethylsilylthio group, octyldimethylsilylthio group, 2-ethylhexyl-dimethylsilylthio group, nonyldimethylsilylthio group, decyldimethylsilylthio group, 3,7-dimethyloctyl-dimethylsilylthio group, lauryldimethylsilylthio group, phenyl-$C_1$-$C_{12}$ alkylsilyloxy group, $C_1$-$C_{12}$ alkoxyphenyl-$C_1$-$C_{12}$ alkylsilylthio group, $C_1$-$C_{12}$ alkylpheny-$C_1$-$C_{12}$ alkylsilylthio group, 1-naphthyl-$C_1$-$C_{12}$ alkylsilylthio group, 2-naphthyl-$C_1$-$C_{12}$ alkylsilylthio group, phenyl-$C_1$-$C_{12}$ alkyldimethylsilylthio group, triphenylsilylthio group, tri-p-xylylsilylthio group, tribenzylsilylthio group, diphenylmethylsilylthio group, t-butyldiphenylsilylthio group and dimethylphenylsilylthio group.

The substituted silylamino group refers to a silylamino group substituted with 1, 2 or 3 groups selected from an alkylamino group, arylamino group, arylalkylamino group and monovalent heterocyclic amino group. The number of carbon atoms thereof is generally about 1 to 60 and preferably 3 to 48. Note that the alkyloxy group, arylamino group, arylalkylamino group and monovalent heterocyclic amino group may have a substituent.

Specific examples thereof include a trimethylsilylamino group, triethylsilylamino group, tripropylsilylamino group, triisopropylsilylamino group, dimethylisopropylsilylamino group, diethylisopropylsilylamino group, t-butylsilyldimethylsilylamino group, pentyldimethylsilylamino group, hexyldimethylsilylamino group, heptyldimethylsilylamino group, octyldimethylsilylamino group, 2-ethylhexyl-dimethylsilylamino group, nonyldimethylsilylamino group, decyldimethylsilylamino group, 3,7-dimethyloctyl-dimethylsilylamino group, lauryldimethylsilylamino group, phenyl-$C_1$-$C_{12}$ alkylsilyloxy group, $C_1$-$C_{12}$ alkoxyphenyl-$C_1$-$C_{12}$ alkylsilylamino group, $C_1$-$C_{12}$ alkylpheny-$C_1$-$C_{12}$ alkylsilylamino group, 1-naphthyl-$C_1$-$C_{12}$ alkylsilylamino group, 2-naphthyl-$C_1$-$C_{12}$ alkylsilylamino group, phenyl-$C_1$-$C_{12}$ alkyldimethylsilylamino group, triphenylsilylamino group, tri-p-xylylsilylamino group, tribenzylsilylamino group, diphenylmethylsilylamino group, t-butyldiphenylsilylamino group and dimethylphenylsilylamino group.

The monovalent heterocyclic group refers to the remaining atomic group obtained by removing a single hydrogen atom from a heterocyclic compound. The number of carbon atoms thereof is generally about 4 to 60 and preferably 4 to 20. Note that the number of carbon atoms of the substituent is not included in the number of those of the heterocyclic group. The heterocyclic compound refers to an organic compound having a ring structure that may have a hetero atom such as oxygen, sulfur, nitrogen, phosphorus or boron other than a carbon atom. Specific examples thereof include a thienyl group, $C_1$-$C_{12}$ alkylthienyl group, pyrrolyl group, furyl group, pyridyl group, $C_1$-$C_{12}$ alkylpyridyl group, piperidyl group, quinolyl group and isoquinolyl group. Of them, thienyl group, $C_1$-$C_{12}$ alkylthienyl group, pyridyl group and $C_1$-$C_{12}$ alkylpyridyl group are preferable.

The heteroaryloxy group generally has about 6 to 60 carbon atoms and preferably 7 to 48 carbon atoms. Specific examples thereof include a thienyloxy group, $C_1$-$C_{12}$ alkoxythienyloxy group, $C_1$-$C_{12}$ alkylthienyloxy group, pyridyloxy group and isoquinolyloxy group. $C_1$-$C_{12}$ alkoxypyridyloxy group and $C_1$-$C_{12}$ alkylpyridyloxy group are preferable.

Specific examples of the $C_1$-$C_{12}$ alkoxy of the $C_1$-$C_{12}$ alkoxypyridyloxy group include methoxy, ethoxy, propyloxy, isopropyloxy, butoxy, isobutoxy, t-butoxy, pentyloxy, hexyloxy, cyclohexyloxy, heptyloxy, octyloxy, 2-ethylhexyloxy, nonyloxy, decyloxy, 3,7-dimethyloctyloxy and lauryloxy.

Specific examples of the $C_1$-$C_{12}$ alkylpyridyloxy group include a methylpyridyloxy group, ethylpyridyloxy group, dimethylpyridyloxy group, propylpyridyloxy group, 1,3,5-trimethylpyridyloxy group, methylethylpyridyloxy group, isopropylpyridyloxy group, butylpyridyloxy group, isobutylpyridyloxy group, t-butylpyridyloxy group, pentylpyridyloxy group, isoamylpyridyloxy group, hexylpyridyloxy group, heptylpyridyloxy group, octylpyridyloxy group, nonylpyridyloxy group, decylpyridyloxy group and dodecylpyridyloxy group.

The heteroarylthio group generally has about 6 to 60 carbon atoms and preferably 7 to 48 carbon atoms. Specific examples thereof include a pyridylthio group, $C_1$-$C_{12}$ alkoxypyridylthio group, $C_1$-$C_{12}$ alkylpyridylthio group and isoquinolylthio group. Of them, a $C_1$-$C_{12}$ alkoxypyridylthio group and $C_1$-$C_{12}$ alkylpyridylthio group are preferable.

The arylalkenyl group generally has about 7 to 60 carbon atoms and preferably 7 to 48 carbon atoms. Specific examples thereof include a phenyl-$C_2$-$C_{12}$ alkenyl group, $C_1$-$C_{12}$ alkoxyphenyl-$C_2$-$C_{12}$ alkenyl group, $C_1$-$C_{12}$alkylphenyl-$C_2$-$C_{12}$ alkenyl group, 1-naphthyl-$C_2$-$C_{12}$ alkenyl group and 2-naphthyl-$C_2$-$C_{12}$ alkenyl group. Of them, a $C_1$-$C_{12}$ alkoxyphenyl-$C_2$-$C_{12}$ alkenyl group and $C_2$-$C_{12}$ alkylphenyl-$C_1$-$C_{12}$ alkenyl group are preferable.

The arylethynyl group generally has about 7 to 60 carbon atoms and preferably 7 to 48 carbon atoms. Specific examples thereof include a phenyl-$C_2$-$C_{12}$ alkynyl group, $C_1$-$C_{12}$ alkoxyphenyl-$C_2$-$C_{12}$ alkynyl group, $C_1$-$C_{12}$ alkylphenyl-$C_2$-$C_{12}$ alkynyl group, 1-naphthyl-$C_2$-$C_{12}$ alkynyl group and 2-naphthyl-$C_2$-$C_{12}$ alkynyl group. Of them, a $C_1$-$C_{12}$ alkoxyphenyl-$C_2$-$C_{12}$ alkynyl group and $C_1$-$C_{12}$ alkylphenyl-$C_2$-$C_{12}$ alkynyl group are preferable.

The substituted carboxyl group generally has about 2 to 60 carbon atoms and preferably 2 to 48 carbon atoms. The substituted carboxyl group refers to a carboxyl group substituted with an alkyl group, aryl group, arylalkyl group or monovalent heterocyclic group. Specific examples thereof include a methoxycarbonyl group, ethoxycarbonyl group,

propoxycarbonyl group, isopropoxycarbonyl group, butoxycarbonyl group, isobutoxycarbonyl group, t-butoxycarbonyl group, pentyloxycarbonyl group, hexyloxycarbonyl group, cyclohexyloxycarbonyl group, heptyloxycarbonyl group, octyloxycarbonyl group, 2-ethylhexyloxycarbonyl group, nonyloxycarbonyl group, decyloxycarbonyl group, 3,7-dimethyl octyloxycarbonyl group, dodecyloxycarbonyl group, trifluoromethoxycarbonyl group, pentafluoroethoxycarbonyl group, perfluorobutoxycarbonyl group, perfluorohexyloxycarbonyl group, perfluorooctyloxycarbonyl group, pyridyloxycarbonyl group, naphthoxycarbonyl group and pyridyloxycarbonyl group. Note that the alkyl group, aryl group, arylalkyl group and monovalent heterocyclic group may have a substituent. The number of carbon atoms of the substituent is not included in the number those of the heterocyclic group.

Examples of the bidentate ligand include, but not particularly limited to, phenylpyridine, phenanthroline and phenylquinoline that may be substituted with an alkyl group and a halogen group ; and bidentate ligands described in JP-A-2003-515897. Specific examples thereof are compounds having the following structural formulas:

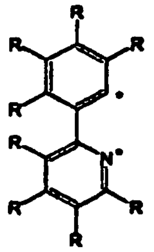
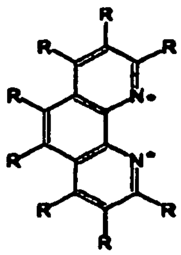
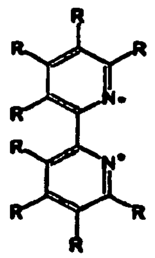
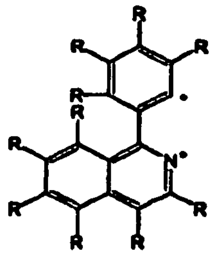
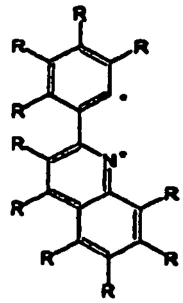

In the structural formula, R represents a hydrogen atom or a substituent. Examples of the substituent include a halogen atom, alkyl group, alkyloxy group, alkylthio group, aryl group, aryloxy group, arylthio group, arylalkyl group, arylalkyloxy group, arylalkylthio group, acyl group, acyloxy group, amide group, acid imido group, imine residue, substituted amino group, substituted silyl group, substituted silyloxy group, substituted silylthio group, substituted silylamino group, a monovalent heterocyclic group, heteroaryloxy group, heteroarylthio group, arylalkenyl group, arylethynyl group, substituted carboxyl group and cyano group. A plurality of Rs may be the same or different.
Reference symbol * in the structural formula indicates a site for binding to a central metal M.

Tridentate ligands may have the following structural formulas:

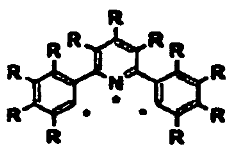
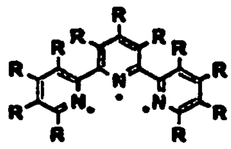
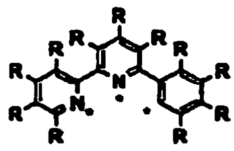
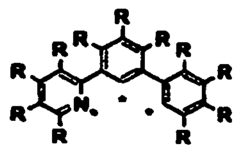
.

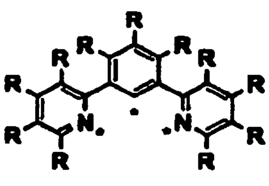
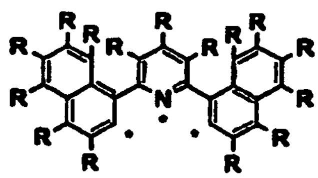
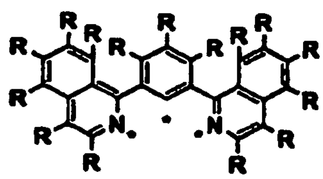

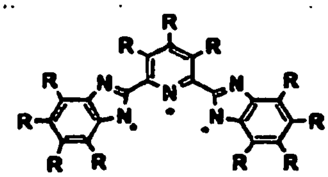
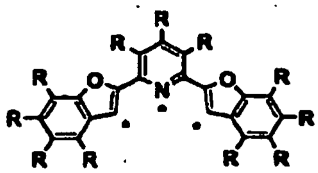
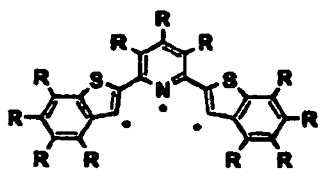

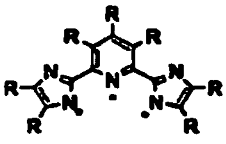 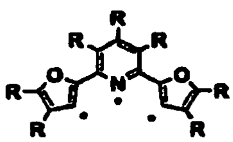 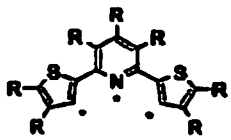 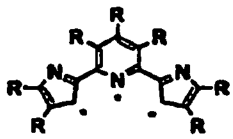

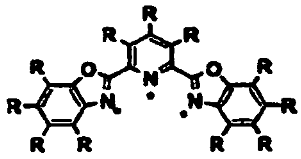 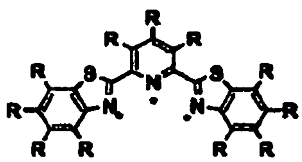

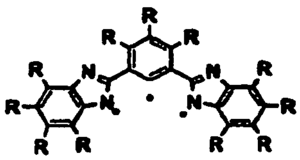 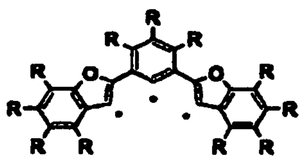 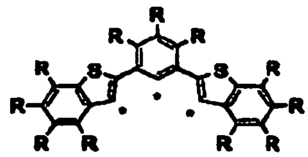

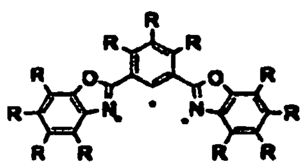 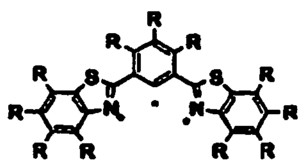

In the structural formulas, R represents a hydrogen atom or a substituent. Examples of the substituent include a halogen atom, alkyl group, alkyloxy group, alkylthio group, aryl group, aryloxy group, arylthio group, arylalkyl group, arylalkyloxy group, arylalkylthio group, acyl group, acyloxy group, amide group, acid imido group, imine residue, substituted amino group, substituted silyl group, substituted silyloxy group, substituted silylthio group, substituted silylamino group, a monovalent heterocyclic group, heteroaryloxy group, heteroarylthio group, arylalkenyl group, arylethynyl group, substituted carboxyl group and cyano group. A plurality of Rs may be the same or different.
Reference symbol * in the structural formulas indicate a site for binding to a central metal M.

Tridentate ligands may have the following structural formulas:

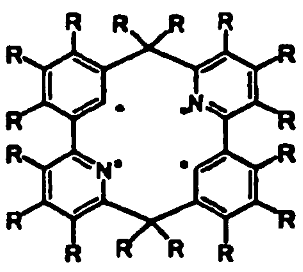 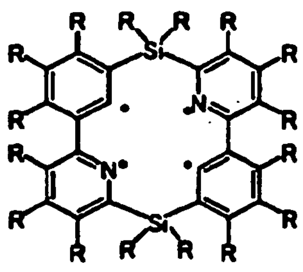 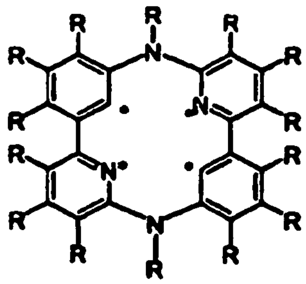

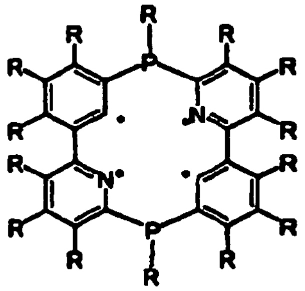
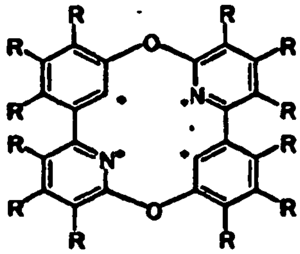
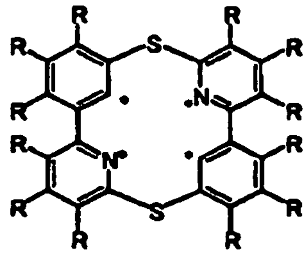

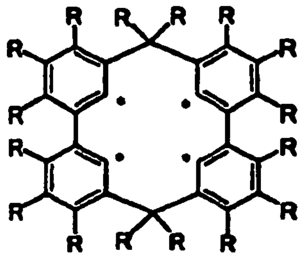
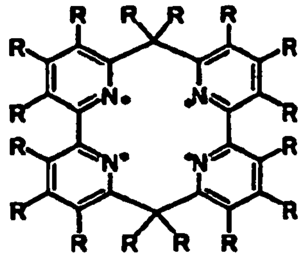

In the structural formulas, R represents a hydrogen atom or a substituent. Examples of the substituent include a halogen atom, alkyl group, alkyloxy group, alkylthio group, aryl group, aryloxy group, arylthio group, arylalkyl group, arylalkyloxy group, arylalkylthio group, acyl group, acyloxy group, amide group, acid imido group, imine residue, substituted amino group, substituted silyl group, substituted silyloxy group, substituted silylthio group, substituted silylamino group, a monovalent heterocyclic group, heteroaryloxy group, heteroarylthio group, arylalkenyl group, arylethynyl group, substituted carboxyl group and cyano group. A plurality of Rs may be the same or different.
Reference symbol * in the structural formulas indicate a site for binding to a central metal M.

A metal complex according to the present invention is preferably a metal complex partially having the structure represented by the general formula (7), (8) (9) or (10) below.

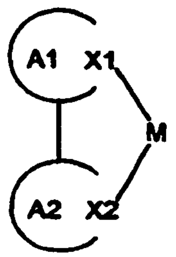

(7)

In the above formula (7), M represents the central metal; A1 ring and A2 ring each independently represent an aromatic ring that may have a substituent; and X1 and X2 present in respective rings each independently represent a ligand atom to the central metal M.

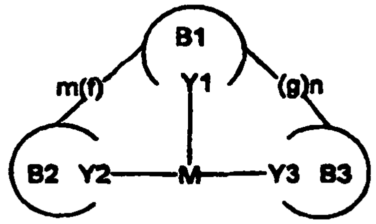

(8)

In the above formula (8), M represents the central metal; B1 ring, B2 ring and B3 ring each independently represent an aromatic ring that may have a substituent; and Y1, Y2 and Y3 present in respective rings each independently represent

a ligand atom to the central metal M. Reference symbols f and g each independently represent an alkylene group having 1 to 6 carbon atoms, an alkenylene group having 1 to 6 carbon atoms or an alkynylene group having 1 to 6 carbon atoms. The carbon atoms of each of the alkylene, alkenylene and alkynylene groups may be substituted with an oxygen atom or a sulfur atom. Reference symbols m and n each independently represent 0 or 1.

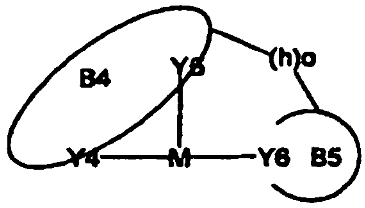

(9)

In the above formula (9), M represents the central metal; B4 ring and B5 ring each independently represent an aromatic ring that may have a substituent; and Y4, Y5 and Y6 present in respective rings each independently represent a ligand atom to the central metal M. Reference symbol h represents an alkylene group having 1 to 6 carbon atoms, an alkenylene group having 1 to 6 carbon atoms or an alkynylene group having 1 to 6 carbon atoms. The carbon atoms of each of the alkylene, alkenylene and alkynylene groups may be substituted with an oxygen atom or a sulfur atom. Reference symbol o represents 0 or 1.

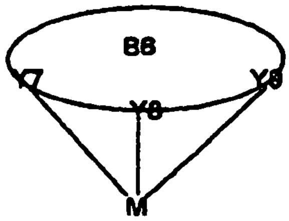

(10)

In the above formula (10), M represents the central metal; B6 ring represents an aromatic ring that may have a substituent; and Y7, Y8 and Y9 present in the ring each independently represent a ligand atom to the central metal M.

Examples of the central metal in the above formulas (7), (8), (9) and (10) include Sc, Ti, V, Cr, Mn, Fe, Co, Ni, Cu, Zn, Y, Zr, Nb, Mo, Tc, Ru, Rh, Pd, Ag, Cd, Hf, Ta, W, Re, Pt, Au and Hg. To obtain high efficiency, Mo, Tc, Ru, Rh, Pd, Ag, W, Re, Pt and Au are preferable; Ru, Rh, Pd, W, Re, Pt and Au are more preferable; Re and Pt are further preferable; and Pt is the most preferable.

In the above formulas (7), (8), (9) and (10), examples of ligand atoms $X_i$ (i=1,2) and $Y_j$ (j=1 to 9) may include a carbon atom, nitrogen atom, oxygen atom, silicon atom, sulfur atom, phosphorus atom, arsenic atom and selenium atom; preferably a carbon atom, nitrogen atom, oxygen atom, silicon atom, sulfur atom, phosphorus atom; further preferably a carbon atom, nitrogen atom, oxygen atom, silicon atom and sulfur atom.

When a metal complex according to the present invention has a tetradentate ligand, the metal complex may preferably contain a partial structure represented by the general formula (4) below.

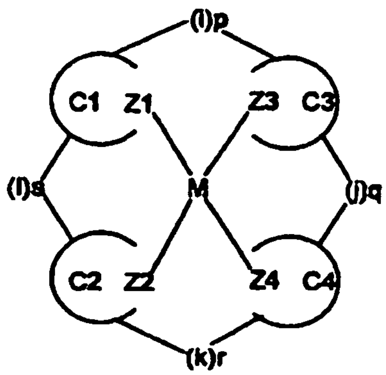

(4)

In the above formula (4), M represents the central metal; C1 ring, C2 ring, C3 ring and C4 ring each independently represent an aromatic ring that may have a substituent; and Z1, Z2, Z3 and Z4 present in respective rings each independently represent a ligand atom to the central metal M. Reference symbols i, j, k and 1 each independently represent an alkylene group having 1 to 6 carbon atoms, an alkenylene group having 1 to 6 carbon atoms or an alkynylene group having 1 to 6 carbon atoms. The carbon atoms of each of the alkylene, alkenylene and alkynylene groups may be substituted with an oxygen atom, sulfur atom, nitrogen atom or phosphorus atom. Reference symbols p, q, r and s each independently represent 0 or 1. Preferably, p, q, r and s each represent 1. Examples of the ligand atom Zk (k=1 to 4) may include a carbon atom, nitrogen atom, oxygen atom, silicon atom, sulfur atom, phosphorus atom, arsenic atom and selenium atom; preferably a carbon atom, nitrogen atom, oxygen atom, sulfur atom, and phosphorus atom; and further preferably, a carbon atom, nitrogen atom, oxygen atom and sulfur atom.

Examples of the central metal in the above formula (4) include Sc, Ti, V, Cr, Mn, Fe, Co, Ni, Cu, Zn, Y, Zr, Nb, Mo, Tc, Ru, Rh, Pd, Ag, Cd, Hf, Ta, W, Re, Pt, Au and Hg. To obtain high efficiency, Mo, Tc, Ru, Rh, Pd, Ag, W, Re, Pt and Au are preferable; Ru, Rh, Pd, W, Re, Pt and Au are more preferable; Re, Pt and Au are further preferable; and Pt is the most preferable.

The metal complex according to the present invention is a metal complex characterized by having a partial structure represented by the general formulas (1-1) below.

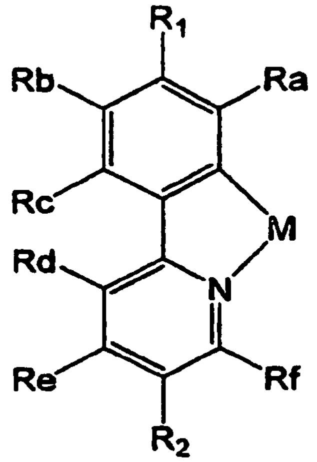

(1-1)

In the general formula (1-1), M represents a central metal; $R_1$ and $R_2$ and Ra to Rf each independently represent a hydrogen atom, alkyl group, alkoxy group, phenyl group or halogen atom. Note that at least one of $R_1$ and $R_2$ is a group represented by the formula (2) below.

In the formula (2), $R_{11}$ to $R_{19}$ each independently represent a hydrogen atom, alkyl group, alkoxy group, phenyl group or a halogen atom. The wave line indicates a binding position.

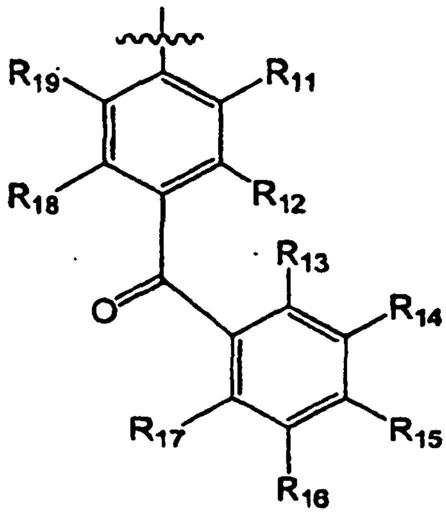

(2)

Examples of the structure may include those shown below:

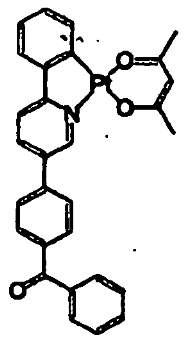
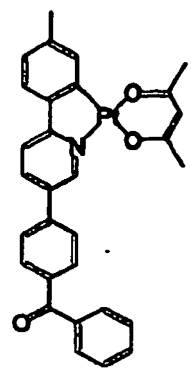
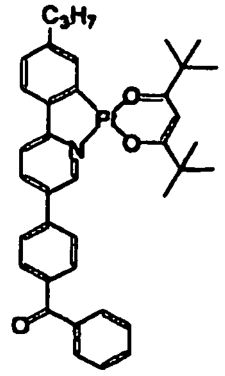
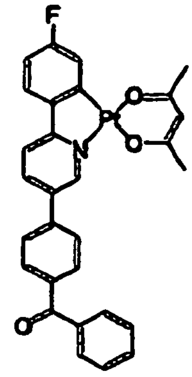
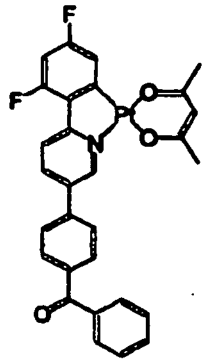
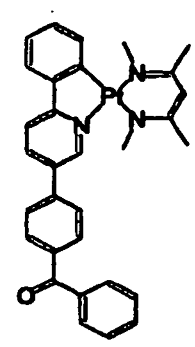
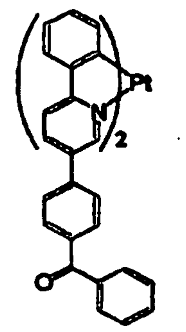
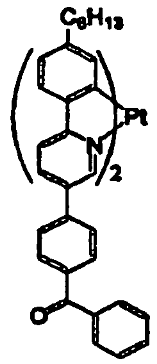
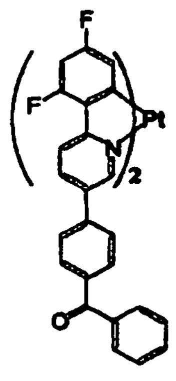
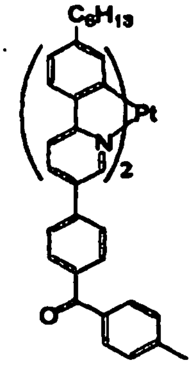
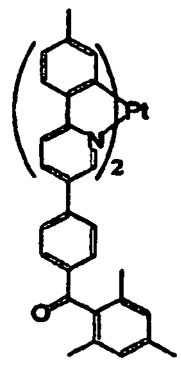
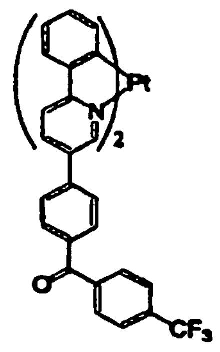
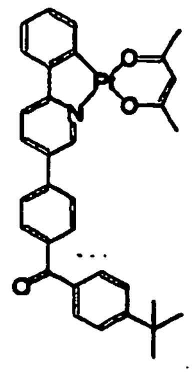
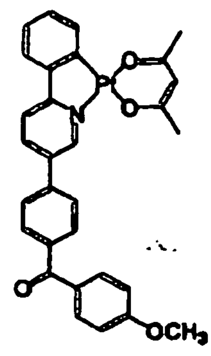
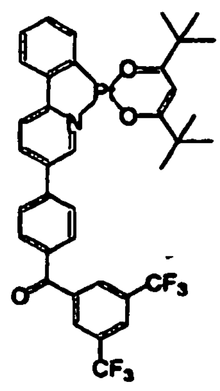
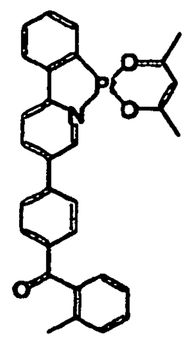

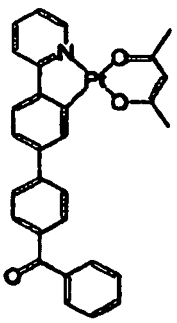

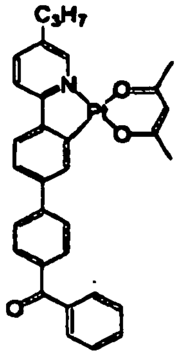

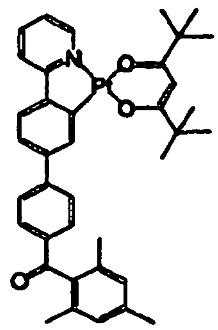

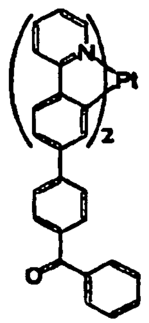

- Method for producing a complex -

A method for synthesizing a metal complex will now be described below.

A metal complex according to the present invention can be produced, for example, by the following method. For example, a 2-phenylpyridine ligand can be synthesized by reacting a compound having a moiety containing a pyridine ring with a compound having a moiety containing a phenyl ring through Suzuki coupling, Grignard coupling using a nickel catalyst or Stille coupling to synthesize a compound serving as a ligand, and reacting the ligand with a desired metal salt in a solution to obtain a complex. In this manner, a metal complex according to the present invention can be synthesized.

More specifically, the compound serving as a ligand can be synthesized by dissolving a compound having a moiety containing a pyridine ring and a compound having a moiety containing a phenyl ring in an organic solvent as needed and reacting the compounds in the presence of an alkali or an appropriate catalyst at a temperature from a melting point to a boiling point (both inclusive) of the organic solvent, for example, in accordance with a method as described in any one of the following documents:
"Organic Reactions", Vol. 14, p.270-490, John Wiley & Sons, Inc., 1965; "Organic Syntheses", Collective Volume VI, p. 407-411, John Wiley & Sons, Inc., 1988; "Chem. Rev.", Vol. 95, p. 2457 (1995); "J. Organomet. Chem.", Vol. 576, p. 147 (1999); "J. Prakt. Chem., Vol. 336, p. 247(1994); "Makromol. Chem., Macromol. Symp.), Vol. 12, p. 229(1987).

The organic solvent to be used for synthesis of a compound serving as a ligand varies depending upon the compounds to be employed and the type of reaction. To suppress a side reaction, the organic solvent to be used is generally subjected to a deoxygenation process in advance and sufficiently treated there. The reaction preferably proceeds under an inert atmosphere. Furthermore, the organic solvent is preferably subjected to a dewatering process in advance except the case where a reaction is performed in a two phase system with water as in the Suzuki coupling reaction.

In synthesizing a compound serving as a ligand, a reaction accelerator such as an alkali or a proper catalyst may be appropriately added to facilitate the reaction. The alkali or proper catalyst can be selected depending upon the reaction to be used; however, they are preferably sufficiently dissolved in the solvent to be used. An alkali or proper catalyst may be mixed with a substrate, for example, by adding the alkali or catalyst gently to a reaction solution (i.e., containing a substrate dissolved or dispersed in an organic solvent) while the reaction solution is stirred under an inert atmosphere such as argon or nitrogen, or conversely, by adding the reaction solution gently to the alkali or catalyst.

In synthesizing a compound serving as a ligand, the reaction temperature is not particularly limited; however, it is generally about -100 to 350°C, preferably 0°C to the boiling point of the solvent to be used. The reaction time is not particularly limited; however, it is usually about 30 minutes to 30 hours.

In synthesizing a compound serving as a ligand, after completion of the aforementioned reaction, a desired product (the compound serving as a ligand) is recovered from the reaction mixture and purified. The recovery and purification methods vary depending upon the resultant compound serving as a ligand. For example, a customary purification method for an organic compound such as recrystallization, sublimation or chromatography may be used.

As a complexation method, that is, a method of reacting a compound serving as a ligand with a metal salt in a solution, for example, to obtain a platinum complex, use is made of the methods described in documents such as Inorg. Chem., 1984, 23, 4249; Chem. Mater. 1999, 11, 3709; Organometallics, 1999, 18, 1801; and Inorg. Chem. 2002, 41, 3055. To obtain a palladium complex, use is made of the method described in a document such as J. Org. Chem., 1987, 52, 73.

The reaction temperature of a complexation reaction is not particularly limited; however, it is generally between the melting point and the boiling point of the solvent to be used, preferably between - 78°C and the boiling point of the

solvent. The reaction time is not particularly limited; however, it is usually about 30 minutes to 30 hours. However, when a microwave reaction apparatus is used in the complexation reaction, the reaction can be performed at not less than the boiling point of the solvent. The reaction time thereof is not particularly limited; however, it is from about several minutes to several hours.

In a complexation reaction, synthesis is performed in accordance with the following operation. A solvent is placed in a flask. While stirring the solvent, the flask is deaerated, for example, by bubbling with an inert gas such as nitrogen gas or argon gas. Thereafter, a metal salt and a compound serving as a ligand are added. The resultant solution is raised in temperature under an inert gas atmosphere while stirring to the temperature at which ligands are exchanged and stirred while maintaining the temperature. The termination of the reaction can be confirmed by detecting stop of raw-material consumption or running out of either one of the raw materials by a TLC monitor or high performance liquid chromatography.

A desired product (metal complex) is recovered and purified from the reaction mixture obtained through the aforementioned reaction. The recovery and purification methods vary depending upon the metal complex. For example, a customary purification method for a complex, such as recrystallization, sublimation or chromatography may be used. To be more specific, the solvent of the reaction mixture is distilled off. Thereafter, the residue is dissolved in an organic solvent such as dichloromethane and purified by silica gel column chromatography to collect solution fractions containing a desired product. The solution is concentrated and an appropriate amount of poor solvent such as methanol is added. The desired product, that is, a metal complex is precipitated by recrystallization, filtrated and dried to obtain the metal complex.

Identification/analysis of a compound can be performed by CHN element analysis, NMR analysis and MS analysis.

The complex of the present invention represented by the following formula (A)

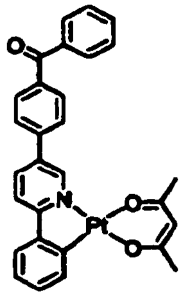

(A)

can be synthesized by the following synthesis route.

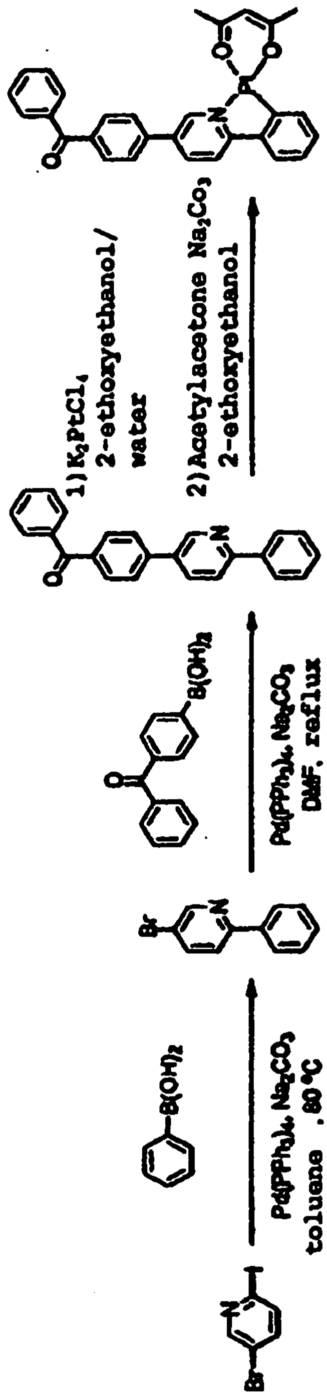


A composition according to the present invention contains a metal complex according to the present invention, and preferably contains the metal complex and a charge transport material.

As the charge transport material of the present invention, an organic compound (low-molecular weight organic compound or polymer) is used. The charge transport materials are classified into hole transport materials and electron transport materials. Examples of the hole transport materials include known hole transport materials so far used in organic EL devices, such as aromatic amines, carbazoles and polyparaphenylene derivatives. Examples of the electron transport materials also include known electron transport materials so far used in organic EL devices. More specifically, mention may be made of metal complexes of an oxadiazole derivative, anthraquinodimethane or a derivative thereof, benzoquinone or a derivative thereof, naphthoquinone or a derivative thereof, anthraquinone or a derivative thereof, tetracyanoanthraquino-dimethane or a derivative thereof, fluorenone derivative, diphenyldicyanoethylene or a derivative thereof, diphenoquinone derivative, or 8-hydroxyquinoline or a derivative thereof. The low molecular weight organic compound of a charge transport material refers to a host compound and charge injection transportable compound used

in low molecular weight organic EL devices. Specific examples thereof are compounds described in "Organic EL Display" (written by Seishi Tokito, Tihaya Adachi, Hideyuki Murata, published by Ohmsha, Ltd.) p. 107; Monthly Display, vol. 9, No. 9, 2003, p. 26-30, JP-A-2004-244400 and 2004-277377. To obtain satisfactory light emission from a metal complex, it is generally preferable that T1 energy of each of these charge transport materials is larger than T1 energy of a metal complex; however, this condition varies depending upon the types of these charge transport materials.

Specific examples of the low molecular weight compound (low molecular weight host compound) include the following compounds.

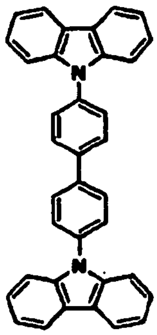
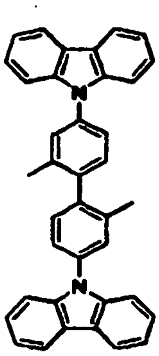
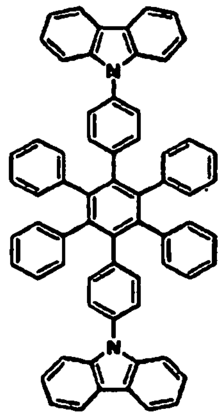
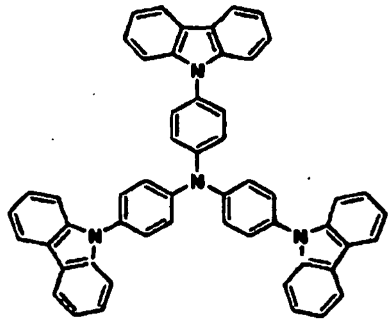

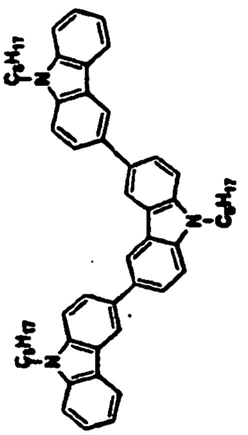

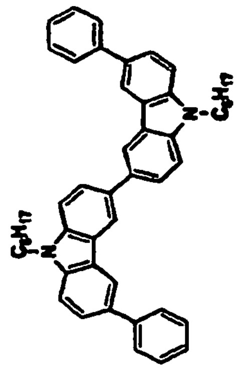

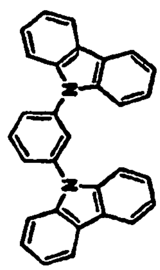

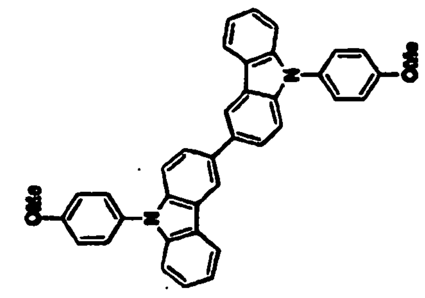

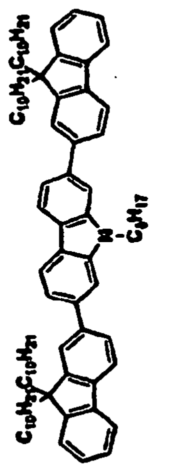

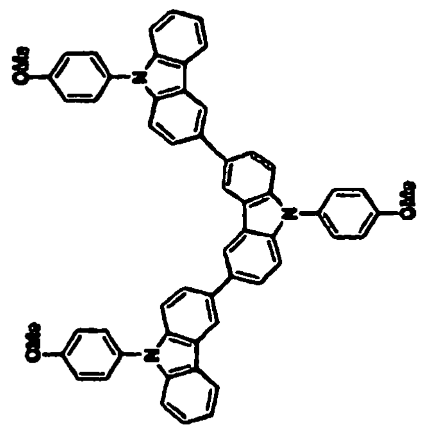

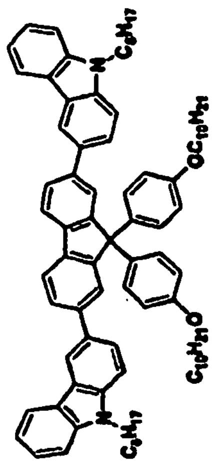

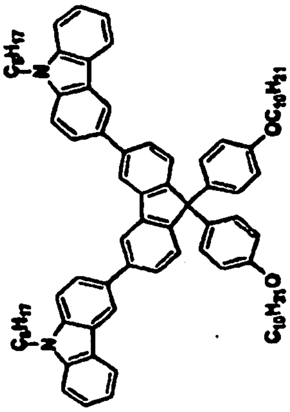

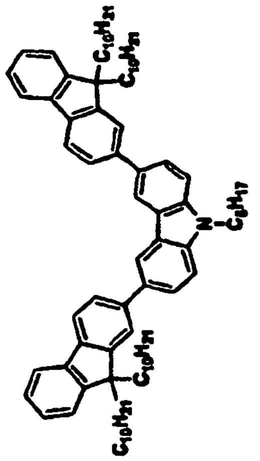

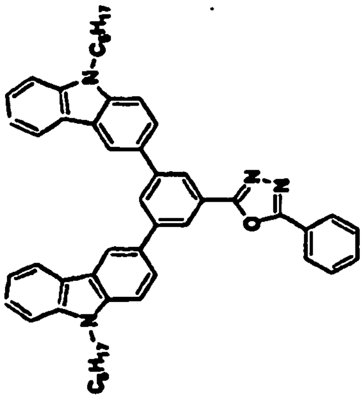

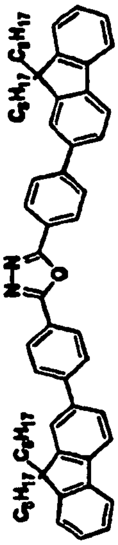

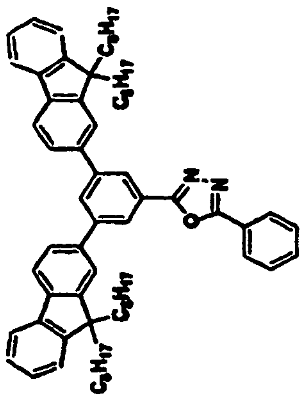

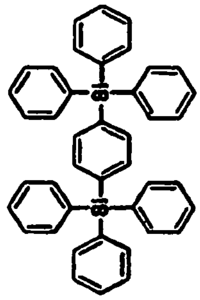

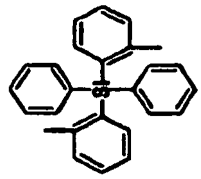

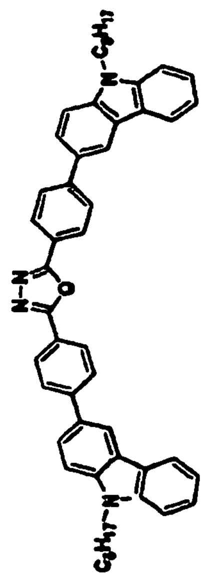

As the charge transport material, a polymer can be used. As the polymer, a non conjugated polymer and a conjugated polymer may be mentioned. As the non conjugated polymer, a polyvinylcarbazole and the like may be mentioned. As the conjugated polymer, a polymer containing an aromatic ring in the main chain may be mentioned. Examples thereof include polymers containing a phenylene group that may have a substituent, fluorene, dibenzothiophene or dibenzofuran, dibenzosilole as a repeat unit in the main chain, and copolymers with these units. Specifically, mention may be made of a polymer compound characterized by having a benzene ring that may have a substituent and/or a partial structure represented by the general formula (2) below. More specifically, mention may be made of the polymers described in JP-A-2003-231741, 2004-059899, 204-002654, 2004-292546, US 5708130, WO 9954385, WO 0046321, WO 02077060; "Organic EL Display" (written by Seishi Tokito, Tihaya Adachi, Hideyuki Murata, published by Ohmsha, Ltd.) p. 111; and Monthly Display, vol. 9, No. 9, 2002, p. 47-51.

Examples of the polymer (polymer host compound) include polymers containing a repeat unit represented by the general formula (6) below.

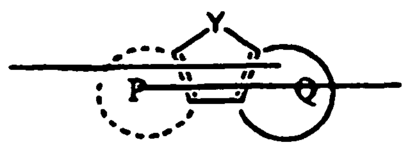

(6)

In the formula, P ring and Q ring each independently represent an aromatic ring; the P ring may not be present. Two bonds are present respectively on the P ring and/or the Q ring when the P ring is present, and present on the 5-membered ring or 6-membered ring each containing Y and/or on the Q ring when the P ring is not present. Furthermore, a substituent may be present on the aromatic ring and/or the 5-membered ring or 6-membered ring containing Y. Y represents -O-, -S-, -Se-, $-B(R_{31})-$, $-N(R_{35})-$, $-C(R_1)(R_2)-$, $-Si(R_1)(R_2)-$, $-P(R_3)-$, $-PR_4(=O)-$, $-C(R_{51})(R_{52})-C(R_{53})(R_{54})-$, $-O-C(R_{55})(R_{36})-$, $-S-C(R_{57})(R_{58})-$, $-N-C(R_{59})(R_{60})-$, $-Si(R_{61})(R_{62})-(R_{63})(R_{64})-$, $-Si(R_{65})(R_{66})-Si(R_{67})(R_{68})-$, $-C(R_{69})=C(R_{70})-$, $-N=C(R_{71})-$ or $-Si(R_{72})=C(R_{73})-$. $R_1$ to $R_4$, $R_{31}$, $R_{35}$ and $R_{51}$ to $R_{73}$ each independently represent a hydrogen atom, alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, silyloxy group, substituted silyloxy group, monovalent heterocyclic group or halogen atom. Of them, an alkyl group, alkoxy group, alkylthio group, aryl group, aryloxy group, arylthio group, arylalkyl group, arylalkoxy group and monovalent heterocyclic group are preferable; an alkyl group, alkoxy group, aryl group and monovalent heterocyclic group are more preferable; and an alkyl group and aryl group are particularly preferable.
Note that specific examples of the alkyl group, alkoxy group, alkylthio group, aryl group, aryloxy group, arylthio group, arylalkyl group, arylalkoxy group, arylalkylthio group, arylalkenyl group, arylalkynyl group, monovalent heterocyclic group and a halogen atom are the same as exemplified above.

As the charge transport material, copolymers and compositions of polymers containing the aforementioned structures.

The ground state energy of a host compound (ESH), the energy of the lowest triplet excited state of a host compound (ETH), the ground state energy of a metal complex (ESMC) and the energy of the lowest excitation triplet state of a metal complex (ETMC) preferably satisfy the relationship:

$$ETH-ESH > ETMC-ESMC-0.2(eV)$$

When a polymer is used in a composition according to the present invention, the number average molecular weight of the polymer in terms of polystyrene is preferably $10^3$ to $10^8$ and further preferably $10^4$ to $10^6$. The weight average molecular weight of the polymer in terms of polystyrene is preferably $10^3$ to $10^8$ and further preferably $5\times10^4$ to $5\times10^6$.

A polymer according to the present invention contains a metal complex according to the present invention in the molecule as a partial structure. More specifically, a metal complex containing a structure represented by the aforementioned general formula (4), (7), (8), (9) or (10) as a partial structure is integrated in the polymer as a partial structure. Furthermore, the polymer having a metal complex according to the present invention in the molecule thereof as a partial structure is preferably a conjugated polymer. As the polymer to which a metal complex is to be integrated, the same polymers as those to be used in a composition according to the present invention as mentioned above may be exemplified. When a metal complex according to the present invention is partially integrated into a polymer, examples of the polymer having the structure of a polymer (A) and the structure of a metal complex (B) in the same molecule may include:

a polymer having the structure of a metal complex (B) in the main chain of the polymer (A);
a polymer having the structure of a metal complex (B) in the terminal of the polymer (A); and
a polymer having the structure of a metal complex (B) in a side chain of the polymer (A).

Examples of the polymers include a polymer containing a partial structure (repeat unit) represented by the general formula (6) and having a number average molecular weight of $10^3$ to $10^8$ in terms of polystyrene and a phosphorescent molecule (metal complex) in a side chain, the main chain and/or the terminal thereof.

The amount of a metal complex in a composition or a polymer according to the present invention is not particularly limited since it varies depending upon the type of organic compound to be used in combination and the property to be optimized; however, generally 0.01 to 80 parts by weight, preferably 0.1 to 60 parts by weight based on the amount of the organic compound (100 parts by weight). Furthermore, at least two types of metal complexes may be contained.

A composition or a polymer of the present invention may contain at lest one type of material selected from the group consisting of a hole transport material, electron transport material and light-emitting material.

As the charge transport material, the aforementioned charge transport materials can be used. As the light-emitting material, a known light-emitting material can be used. As a low molecular weight compound, for example, use may be made of a naphthalene derivative, anthracene or a derivative thereof, perylene or a derivative thereof, pigments such as a polymethine based, xanthene based, coumarin based and cyanine based pigments, a metal complex of 8-hydroxyquinoline or a derivative thereof, aromatic amine, tetraphenylcyclopentadiene or a derivative or a tetraphenylbutadiene or a derivative thereof.

An ink composition according to the present invention is characterized by containing a metal complex, composition or polymer according to the present invention as mentioned above.

Any ink composition may be used as long as it contains at least one type of metal complex according to the present invention. The ink composition may contain not only the metal complex of the invention but also a hole transport material, electron transport material, light-emitting material, solvent and additives such as a stabilizer.

The ratio of solid matter including a metal complex of the present invention in the ink composition is generally 20 wt % to 100 wt % and preferably 40 wt % to 100 wt % relative to the total weight of the composition excluding the solvent.

When a solvent is contained in an ink composition, the ratio of the solvent is 1 wt % to 99.9 wt %, preferably 60 wt % to 99.5 wt %, and further preferably, 80 wt % to 99.0 wt %, relative to the total weight of the composition.

The viscosity of the ink composition varies depending upon the printing method. When an ink composition is passed through an ejection apparatus as is in the case of inkjet printing, the viscosity at 25°C preferably falls within the range of 1 to 20 mPa·s in order to prevent clogging of ejection nozzles and spray liquid droplets from flying away from a right direction.

An ink composition according to the present invention (hereinafter, simply refers to as "the solution" or "the solution of the invention") may contain not only a metal complex or a composition according to the present invention but also additives for controlling viscosity and/or surface tension. Examples of the additives may include a polymer compound (thickner) of a high molecular weight or a poor solvent for increasing viscosity, a compound of a low molecular weight for decreasing viscosity, and a surfactant for reducing surface tension. These additives may be used in an appropriate combination.

As the polymer compound (thickner) of a high molecular weight, any polymer may be used as long as it can be dissolved in the same solvent that dissolves a metal complex or composition according to the present invention and unless it blocks emission or charge transport. For example, polystyrene and polymethylmethacrylate of a high molecular weight can be used. The weight average molecular weight thereof is preferably 500,000 or more and more preferably 1,000,000 or more.

A poor solvent may be used as a thickner. Viscosity can be increased by adding a small amount of poor solvent for the solid matter to the solution. When a poor solvent is added for this purpose, the type and addition amount of solvent may be selected within the range where no solid matter precipitates in the solution. In consideration of storage stability, the amount of poor solvent is preferably 50 wt % or less and further preferably 30 wt % or less relative to the entire amount of the solution.

An ink composition according to the present invention may further contain an antioxidant to improve storage stability. As the antioxidant, any antioxidant may be used as long as it can be solved in the same solvent that dissolves a metal complex or composition according to the present invention and unless it blocks light emission or charge transport.

A solvent to be used for forming a film from a solution preferably dissolves or uniformly disperse the components of, for example, a composition according to the present invention. Examples of the solvent include chlorine based solvents such as chloroform, methylene chloride, 1,2-dichloroethane, 1,1,2-trichloroethane, chlorobenzene and o-dichlorobenzene; ether based solvents such as tetrahydrofuran and dioxane; aromatic hydrocarbon based solvents such as toluene and xylene; aliphatic hydrocarbon based solvents such as cyclohexane, methylcyclohexane, n-pentane, n-hexane, n-heptane, n-octane, n-nonane and n-decane; ketone based solvents such as acetone, methylethylketone and cyclohexanone; ester based solvents such as ethyl acetate, butyl acetate and ethyl cellosolve acetate; polyhydric alcohols and derivatives thereof such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin and 1,2-hexane diol; alcohol based solvents such as methanol, ethanol, propanol, isopropanol and cyclohexanol; sulfoxide based solvents such as dimethylsulfoxide; amido based solvents such as N-methyl-2-pyrrolidone and N,N-dimethylformamide.

These organic solvents may be used singly or in combination of two or more. At least one type of organic solvent selecting from the aforementioned solvents, having at least one benzene ring, a melting point of 0°C or less and a boiling point of 100°C or more is preferably contained.

As the type of solvent, an aromatic hydrocarbon based solvent, aliphatic hydrocarbon based solvent, ester based solvent and ketone based solvent are preferable in view of solubility to an organic solvent, uniformity of a formed film, and viscosity. Preferable examples of the solvent include toluene, xylene, ethylbenzene, diethylbenzene, trimethylbenzene, n-propylbenzene, isopropylbenzene, n-butylbenzene, isobutylbenzene, s-butylbenzene, anisole, ethoxybenzene, 1-methylnaphthalene, cyclohexane, cyclohexanone, cyclohexylbenzene, bicyclohexyl, cyclohexenylcyclohexanone, n-heptylcyclohexane, n-hexylcyclohexane, 2-propylcyclohexanone, 2-heptanon,3-heptanon, 4-heptanon, 2-octanone, 2-nonanon, 2-decanone and dicyclohexyl ketone. More preferably, at least one type of solvent of xylene, anisole, cyclohexylbenzene and bicyclohexyl may be contained.

The number of types of solvents used in the solution is at least 2 or more preferably, 2 to 3, and further preferably, 2, in view of film-formability and device characteristics,

When at least 2 types of solvents are contained in the solution, the solvent having the highest boiling point is preferably contained in an amount of 40 to 90 wt %, more preferably, 50 to 90 wt %and further preferably 65 to 85 wt % based on the weight of the all solvents contained in the solution, in view of viscosity and film-formability.

An ink composition according to the present invention preferably has a viscosity of 1 to 100 mPa·s at 25°C.

A polymer or a composition containing a metal complex according to the present invention can be used not only as a light-emitting material but also as an organic semiconductor material, optical material, or a conductive material by doping. Therefore, a light emitting thin film, conductive thin film, organic semiconductor thin film, or the like can be formed using the metal complex, polymer or composition.

Next, a photoelectric device according to the present invention will be described. The photoelectric device of the present invention is characterized by having a layer containing a metal complex or a composition according to the present invention between the electrodes consisting of an anode and a cathode, and used, for example, as a light-emitting device, switching device and photoelectric conversion device. When the device is used as a light-emitting device, the layer containing a metal complex according to the present invention preferably serves as a light emitting layer.

A photoelectric device according to the present invention may contain a charge transport layer or a charge blocking layer between the electrodes consisting of an anode and a cathode. The charge transport layer herein refers to a hole transport layer or an electron transport layer. The charge blocking layer refers to a hole blocking layer or an electron blocking layer. Examples thereof include a light-emitting device having an electron transport layer or a hole blocking layer between a cathode and a photoelectric layer; a light-emitting device having a hole transport layer or an electron blocking layer between an anode and a photoelectric layer; and a light-emitting device having an electron transport layer or a hole blocking layer between a cathode and a photoelectric layer as well as a hole transport layer or an electron block layer between an anode and the photoelectric layer. The functions of the electron transport layer and hole blocking layer used herein are the same. Both layers can be formed of the same material, as described in "All about organic EL". p.162 (written by Junji Shiroto, Nippon Jitsugyo Publishing Co., Ltd.). Either one of the functions is sometimes strongly expressed depending upon the characteristics of the material. The same feature as this may be said with respect to the hole transport layer and electron blocking layer. The device structure of a light-emitting device according to the present invention is, for example, described in Patent Document (Journal of the SID 11/1, 161-166, 2003).
Additional examples thereof include a light-emitting device having a layer containing a conductive polymer between at least one of the electrodes and a photoelectric layer and in adjacent to the electrode; and a light-emitting device having a buffer layer having an average thickness of 2 nm or less between at least one of the electrodes and a photoelectric layer and in adjacent to the electrode.

More specifically, the following structures (a) to (d) may be mentioned.

a) Anode/photoelectric layer/cathode
b) Anode/hole transport layer/photoelectric layer/cathode
c) Anode/ photoelectric layer/electron transport layer/cathode
d) Anode/hole transport layer/photoelectric layer/electron transport layer/cathode
(Symbol "/" means that individual layers are laminated in adjacent to each other. The same definition will be employed hereinafter.)

The photoelectric layer is a layer having a photoelectric function, in other words, a thin film having luminosity, conductivity or photoelectric conversion function. The hole transport layer is a layer having a function of transporting holes. The electron transport layer is a layer having a function of transporting electrons. Note that the electron transport layer and hole transport layer are collectively called as a charge transport layer. The number of photoelectric layers, hole transport layers and electron transport layers may be each independently 2 or more.

Of the charge transport layers provided in adjacent to an electrode, the layer having a function of improving charge injection efficiency from the electrode, thereby effectively reducing the drive voltage of a device is generally called particularly as a charge injection layer (hole injection layer or electron injection layer).

To improve adhesion to an electrode and to improve charge injection from the electrode, a charge injection layer as mentioned above or an insulating layer having a thickness of 2 nm or less may be provided in adjacent to the electrode. Alternatively, for improving the adhesion to the interface, preventing contamination and for other purpose, a thin buffer layer may be inserted between the charge transport layer and the photoelectric layer.

Furthermore, a hole blocking layer may be inserted between the interface with the photoelectric layer in order to transport electrons and confine holes.

The laminate order, number, and thickness of layers may be appropriately set in view of luminous efficiency

and the working life of a device.

In the present invention, examples of a light-emitting device having a charge injection layer (electron injection layer and hole injection layer) include a light-emitting device having a charge injection layer in adjacent to a cathode; and a light-emitting device having a charge injection layer in adjacent to an anode.

Specific examples thereof are light-emitting devices having the following structures (e) to (p).

e) anode/charge injection layer/photoelectric layer/cathode
f) anode/photoelectric layer/charge injection layer/cathode
g) anode/charge injection layer/photoelectric layer/charge injection layer/cathode
h) anode/charge injection layer/hole transport layer/photoelectric layer/cathode
i) anode/hole transport layer/photoelectric layer/charge injection layer/cathode
j) anode/charge injection layer/hole transport layer/photoelectric layer/charge injection layer/cathode
k) anode/charge injection layer/photoelectric layer/charge transport layer/cathode
l) anode/photoelectric layer/electron transport layer/charge injection layer/cathode
m) anode/charge injection layer/photoelectric layer/electron transport layer/charge injection layer/cathode
n) anode/charge injection layer/hole transport layer/photoelectric layer/charge transport layer/cathode
o) anode/hole transport layer/photoelectric layer/electron transport layer/charge injection layer/cathode
p) anode/charge injection layer/hole transport layer/photoelectric layer/electron transport layer/charge injection layer/cathode

Specific examples of the charge injection layer include a layer containing a conductive polymer; a layer provided between an anode and a hole transport layer and containing a material having an ionization potential, which is a medium value between that of an anode material and that of a hole transport material contained in the hole transport layer; and a layer provided between a cathode and an electron transport layer and containing a material having an electron affinity, which is a medium value between that of a cathode material and that of an electron transport material contained in the electron transport layer.

When the charge injection layer is the layer containing a conductive polymer, the electric conductivity of the conductive polymer is preferably from $10^{-5}$S/cm to $10^{3}$S/cm (both inclusive). To reduce leak current between light emitting pixels, the electric conductivity is preferably from $10^{-5}$S/cm to $10^{2}$S/cm (both inclusive), and further preferably, from $10^{-5}$S/cm to $10^{1}$S/cm (both inclusive).

To set the electric conductivity of the conductive polymer at $10^{-5}$S/cm to $10^{3}$S/cm (both inclusive), generally an appropriate amount of ions is doped into the conductive polymer.

The type of ion to be doped into a hole injection layer is anion and cation to an electron injection layer. Examples of the anion include polystyrene sulfonate ions, alkylbenzene sulfonate ions and camphor sulfonate ions. Examples of the cation include lithium ions, sodium ions, potassium ions and tetrabutylammonium ions.

The film thickness of the charge injection layer is, for example, 1 nm to 100 nm and preferably 2 nm to 50 nm.

The material to be used in the charge injection layer may be appropriately selected in consideration of the materials used in the electrode and the layer adjacent thereto. Examples thereof include polyaniline and a derivative thereof, a polyaminophene and a derivative thereof, polypyrrole and a derivative thereof, polyphenylenevinylene and a derivative thereof, polythienylenevinylene and a derivative thereof, polyquinoline and a derivative thereof, polyquinoxaline and a derivative thereof, electroconductive polymers such as a polymer containing an aromatic amine structure in the main chain or a side chain thereof, metallophthalocyanine (e.g. copper phthalocyanine) and carbon.

The insulating layer having a film thickness of 2 nm or less has a function of facilitating charge injection. Examples of the material for the insulating layer include a metal fluoride, metal oxide and organic insulating material. Examples of the light-emitting device having an insulating layer having a film thickness of 2 nm or less include a light-emitting device, which has an insulating layer having a film thickness of 2 nm or less in adjacent to a cathode, and a light-emitting device, which has an insulating layer having a film thickness of 2 nm or less in adjacent to an anode.

Specific examples thereof are light-emitting devices having the following structures q) to ab).

q) anode/insulating layer of ≤ 2 nm thickness/photoelectric layer/cathode
r) anode/photoelectric layer/insulating layer of ≤ 2 nm thickness/cathode
s) anode/insulating layer of ≤ 2 nm thickness/photoelectric layer/insulating layer of ≤ 2 nm thickness/cathode
t) anode/insulating layer of ≤ 2 nm thickness/hole transport layer/photoelectric layer/cathode
u) anode/hole transport layer/photoelectric layer/insulating layer of ≤ 2 nm thickness/cathode
v) anode/insulating layer of ≤ 2 nm thickness/hole transport layer/photoelectric layer/insulating layer of ≤ 2 nm thickness/cathode
w) anode/insulating layer of ≤ 2 nm thickness/photoelectric layer/electron transport layer/cathode
x) anode/photoelectric layer/electron transport layer/insulating layer of ≤ 2 nm thickness/cathode

y) anode/insulating layer of ≤ 2 nm thickness/photoelectric layer/electron transport layer/insulating layer of ≤ 2 nm thickness/cathode
z) anode/insulating layer of ≤ 2 nm thickness/hole transport layer/photoelectric layer/electron transport layer/cathode
aa) anode/hole transport layer/photoelectric layer/electron transport layer/insulating layer of ≤ 2 nm thickness/cathode
ab) anode/insulating layer of ≤ 2 nm thickness/hole transport layer/photoelectric layer/electron transport layer/insulating layer of ≤ 2 nm thickness/cathode.

The hole blocking layer has a function of transferring electrons and confining holes transported from an anode, provided on the interface of a photoelectric layer facing the cathode and composed of a material having a larger ionization potential than that of the photoelectric layer, such as Bathocuproine or a metallic complex of 8-hydroxyquinoline or a derivative thereof.
The film thickness of the hole blocking layer is, for example, 1 nm to 100 nm and preferably 2 nm to 50 nm.
Specific examples thereof are light-emitting devices having the following structures ac) to an).

ac) anode/charge injection layer/photoelectric layer/hole blocking layer/cathode
ad) anode/photoelectric layer/hole blocking layer/charge injection layer/cathode
ae) anode/charge injection layer/photoelectric layer/hole blocking layer/charge injection layer/cathode
af) anode/charge injection layer/hole transport layer/photoelectric layer/hole blocking layer/cathode
ag) anode/hole transport layer/photoelectric layer/hole blocking layer/charge injection layer/cathode
ah) anode/charge injection layer/hole transport layer/photoelectric layer/hole blocking layer/charge injection layer/cathode
ai) anode/charge injection layer/photoelectric layer/hole blocking layer/charge transport layer/cathode
aj) anode/photoelectric layer/hole blocking layer/electron transport layer/charge injection layer/cathode
ak) anode/charge injection layer/photoelectric layer/hole blocking layer/electron transport layer/charge injection layer/cathode
al) anode/charge injection layer/hole transport layer/photoelectric layer/hole blocking layer/charge transport layer/cathode
am) anode/hole transport layer/photoelectric layer/hole blocking layer/electron transport layer/charge injection layer/cathode
an) anode/charge injection layer/hole transport layer/photoelectric layer/hole blocking layer/electron transport layer/charge injection layer/cathode

In manufacturing a photoelectric device according to the present invention, a film of a photoelectric material including a charge transport material is formed from a solution. In this case, the film can be formed just by applying the solution and removing the solvent by dehydration. In the case where a charge transport material and light-emitting material are mixed, the same manner can be applied. This is very advantageous in manufacturing. Examples of the method for forming a film from a solution include coating methods such as spin coat method, casting method, microgravure coat method, gravure coat method, bar coat method, roll coat method, wire-bar coat method, dip coat method, spray coat method, screen printing, flexographic printing method, offset printing method and inkjet printing method. When a light-emitting material including a charge transport material has a relative low molecular weight, a photoelectric layer may be formed by a vacuum deposition method.
In a light-emitting device according to the present invention, a light-emitting material other than a photoelectric material according to the present invention may be used in combination in the photoelectric layer, that is, a light emitting layer. Furthermore, in the photoelectric device of the present invention, the light emitting layer containing the light-emitting material other than a photoelectric material according to the present invention is laminated with a photoelectric layer containing a light-emitting material according to the present invention.
As the light-emitting material, a known compound may be used. As the known compound having a low molecular weight, for example, use may be made of a naphthalene derivative, anthracene or a derivative thereof, perylene or a derivative thereof, pigments such as polymethine based, xanthene based, coumarin based and cyanine based pigments, a metal complex of 8-hydroxyquinoline or a derivative thereof, aromatic amine, tetraphenylcyclopentadiene or a derivative thereof, or tetraphenyl butadiene or a derivative thereof.
More specifically, the known compounds such as those described in JP-A-57-51781 and 59-194393 may be used.
When a photoelectric device according to the present invention has a hole transport layer, as an example of the hole transport material to be used, use may be made of polyvinylcarbazole or a derivative thereof, polysilane or a derivative thereof, a polysiloxane derivative having an aromatic amine in a side chain thereof or the main chain, a pyrazoline derivative, an arylamine derivative, a stilbene derivative, a triphenyldiamine derivative, polyaniline or a derivative thereof, polyaminofen or a derivative thereof, polypyrrole or a derivative thereof, poly(p-phenylenevinylene) or a derivative thereof, or poly(2,5-thienylenevinylene) or a derivative thereof.

Specific examples of the hole transport material include those described in JP-A-63-70257, 63-175860, 2-135359, 2-135361, 2-209988, 3-37992 and 3-152184.

Of them, as a hole transport material to be used in the hole transport layer, for example, use may be preferably made of polymer hole transport materials such as polyvinylcarbazole or a derivative thereof, polysilane or a derivative thereof, a polysiloxane derivative having an aromatic amine compound group in a side chain or the main chain thereof, polyaniline or a derivative thereof, polyaminofen or a derivative thereof, poly(p-phenylenevinylene) or a derivative thereof, or poly(2,5-thienylenevinylene) or a derivative thereof; and more preferably, polyvinylcarbazole or a derivative thereof, or polysilane or a derivative thereof, a polysiloxane derivative having an aromatic amine compound group in a side chain or the main chain thereof. In the case of a low molecular weight hole transport material, it is preferably used by dispersing it in a polymer binder.

The polyvinylcarbazole or a derivative thereof can be obtained from a vinyl monomer by cation polymerization or radical polymerization.

Examples of the polysilane or a derivative thereof include the compounds described in Chem. Rev. Vol. 89, p. 1359 (1989) and the published specification of British Patent GB 2300196. They can be synthesized by the methods described in these documents. In particular, a kipping method is suitably used.

Since a siloxane skeleton structure has virtually no transportability of holes, polysiloxane or a derivative thereof having a structure of the low molecular weight hole transporting material in a side chain or the main chain thereof is suitably used. In particular, mention is made of polysiloxane or a derivative thereof having an aromatic amine in a side chain or the main chain thereof.

A method for forming a film of a hole transport layer is not particularly limited; however, when a low-molecular weight hole transport material is used, a method for forming a film from a solution mixture containing a polymer binder is exemplified. When a high molecular weight hole transport material is used, a method for forming a film from a solution is exemplified.

The solvent to be used in forming a film from a solution is not particularly limited as long as it can dissolve a hole transport material. Examples of the solvent include chlorine based solvents such as chloroform, methylene chloride and dichloroethane; ether based solvents such as tetrahydrofuran; aromatic hydrocarbon based solvents such as toluene and xylene; ketone based solvents such as acetone and methylethyl ketone; and ester based solvents such as ethyl acetate and butyl acetate and ethylcellosolve acetate.

Examples of the method for forming a film from a solution include coating methods such as a spin coat method, casting method, microgravure coat method, gravure coat method, bar coat method, roll coat method, wire-bar coat method, dip coat method, spray coat method, screen printing, flexographic printing method, offset printing method and inkjet printing method.

As the polymer binder to be mixed, one that cannot extremely block charge transport is preferably used and one whose absorption for visible light is low is suitably used. Examples of the polymer binder include polycarbonate, polyacrylate, polymethylacrylate, polymethylmethacrylate, polystyrene, polyvinylchloride and polysiloxane.

The most suitable film thickness of a hole transport layer varies depending upon the material to be used and may be chosen so as to obtain an appropriate drive voltage and luminous efficiency. The hole transport layer must have an appropriate thickness so that formation of a pin hole is at least prevented. When the film is excessively thick, the drive voltage of the device undesirably increases. Accordingly, the film thickness of the hole transport layer is, for example, from 1 nm to 1 $\mu$m, preferably 2 nm to 50 nm, and further preferably, 5 nm to 200 nm.

When a photoelectric device according to the present invention has an electron transport layer, a known electron transport material may be used. As an example, use may be made of a metal complex of an oxadiazole derivative, anthraquinodimethane or a derivative thereof, benzoquinone or a derivative thereof, naphthoquinone or a derivative thereof, anthraquinone or a derivative thereof, tetracyanoanthraquinodimethane or a derivative thereof, a fluorenone derivative, diphenyldicyanoethylene or a derivative thereof, a diphenoquinone derivative, or 8-hydroxyquinoline or a derivative thereof; polyquinoline or a derivative thereof; polyquinoxaline or a derivative thereof; or polyfluorene or a derivative thereof.

Specific examples of the electron transport material include those described, for example, in JP-A-63-70257, 63-175860, 2-135359, 2-135361, 2-209988, 3-37992 and 3-152184.

Of them, a metal complex of an aminoxadiazole derivative, benzoquinone or a derivative thereof, anthraquinone or a derivative thereof, or 8-hydroxyquinoline or a derivative thereof; polyquinoline or a derivative thereof; polyquinoxaline or a derivative thereof; polyfluorene or a derivative thereof is preferable; and 2-(4-biphenylyl)-5-(4-t-butylphenyl)-1,3,4-oxadiazole, benzoquinone, anthraquinone, tris(8-quinolinol)aluminum or polyquinoline is further preferable.

A method of forming a film of an electron transport layer is not particularly limited; however, when a low-molecular weight electron transport material is used, a vacuum deposition method for forming a film from powder or a method of forming a film from a solution or a molten state is exemplified. When a high molecular weight electron transport material

is used, a method of forming a film from a solution or a molten state is exemplified. When a film formed from a solution or a molten state, a polymer binder may be used in combination.

The solvent to be used in forming a film from a solution is not particularly limited as long as it can dissolve an electron transport material and/or a polymer binder. Examples of the solvent include chlorine based solvents such as chloroform, methylene chloride and dichloroethane; ether based solvents such as tetrahydrofuran; aromatic hydrocarbon based solvents such as toluene and xylene; ketone based solvents such as acetone and methylethyl ketone; and ester based solvents such as ethyl acetate and butyl acetate and ethylcellosolve acetate.

Examples of the method for forming a film from a solution or a molten state include coating methods such as a spin coat method, casting method, microgravure coat method, gravure coat method, bar coat method, roll coat method, wire-bar coat method, dip coat method, spray coat method, screen printing, flexographic printing method, offset printing method and inkjet printing method.

As the polymer binder to be mixed, one that cannot extremely block charge transport is preferable and one whose absorption of light is low is suitably used. As an example of the polymer binder, use may be made of poly(N-vinylcarbazole), polyaniline or a derivative thereof, polyaminofen or a derivative thereof, poly(p-phenylenevinylene) or a derivative thereof, poly(2,5-thienylenevinylene) or a derivative thereof, polycarbonate, polyacrylate, polymethylacrylate, polymethylmethacrylate, polystyrene, polyvinylchloride or polysiloxane.

The most suitable film thickness of an electron transport layer varies depending upon the material to be used and may be chosen so as to obtain an appropriate drive voltage and luminous efficiency. The electron transport layer must have an appropriate thickness so that formation of a pin hole is at least prevented. When the film is excessively thick, the drive voltage of the device undesirably increases. Accordingly, the film thickness of the electron transport layer is, for example, from 1 nm to 1 μm, preferably 2 nm to 50 nm, and further preferably, 5 nm to 200 nm.

As a substrate on which a photoelectric device according to the present invention is to be formed, any substrate may be used as long as it is not affected when electrode and individual layers of the photoelectric device are formed. Examples of the substrate include glass, plastic, polymer film and silicon substrates. When an opaque substrate is used, the electrode placed in opposite thereto is preferably transparent or translucent.

Generally, at least one of the electrodes consisting of an anode and a cathode is transparent or translucent, and more preferably, the anode is transparent or translucent.

As the material for the anode, a conductive metal oxide film or a translucent metal thin film is used. Specific examples include indium oxide, zinc oxide, tin oxide and an indium/tin/oxide (ITO), which is a complex of these, and film (NESA) formed of an electroconductive glass of an indium/zinc/oxide and the like, gold, platinum, silver and copper. Of them, ITO, indium/zinc/oxide and tin oxide are preferable.. Examples of the film formation method include a vacuum deposition method, sputtering method, ion plating method and plating method. Furthermore, as the anode, use may be made of a transparent electroconductive film made of an organic compound such as polyaniline or a derivative thereof, or a polyaminofen or a derivative thereof.

The film thickness of the anode can be appropriately selected in consideration of light permeability and electroconductivity. The film thickness is, for example, 10 nm to 10 μm, preferably 20 nm to 1 μm and further preferably, 50 nm to 500 nm.

To facilitate charge injection into the anode, a layer formed of a phthalocyanine derivative, electroconductive polymer or carbon, or a layer having an average thickness of 2 nm or less and formed of a metal oxide, metal fluoride or organic insulating material may be provided.

As the material for a cathode to be used in a light-emitting device according to the present invention, a material having a small work function is preferable. Examples thereof include metals such as lithium, sodium, potassium, rubidium, cesium, beryllium, magnesium, calcium, strontium, barium, aluminum, scandium, vanadium, zinc, yttrium, indium, cerium, samarium, europium, terbium and ytterbium; alloys formed of two or more types of metals selected from these, alloys formed of at least one type of metals selected from these and at least one type of metal selected from gold, silver, platinum, copper, manganese, titanium, cobalt, nickel, tungsten and tin; and graphite or a graphite interlayer compound. Examples of the alloys include magnesium-silver alloy, magnesium-indium alloy, magnesium-aluminum alloy, indium-silver alloy, lithium-aluminum alloy, lithium-magnesium alloy, lithium-indium alloy and calcium-aluminium alloy. A laminate structure formed of two or more layers may be used as a cathode..

The film thickness of the cathode may be appropriately selected in consideration of electroconductivity and durability. The film thickness is, for example, from 10 nm to 10 μm, preferably 20 nm to 1 μm, and further preferably, 50 nm to 500 nm.

As the film formation method for a cathode, use may be made of a vacuum deposition method, sputtering method or laminate method employing thermocompression bonding of metal thin films. Furthermore, a layer formed of an electroconductive polymer or a layer having an average thickness of 2 nm or less and formed of a metal oxide, metal fluoride or organic insulating material may be provided between the cathode and an organic compound layer. Alternatively, after the cathode is formed, a protective layer for protecting the light-emitting device may be applied. To use the light-emitting device stably for a long time, a protective layer and/or a protection cover may be applied in order to protect the

device from the outside world.

As the protective layer, use may be made of a polymer compound, metal oxide, metal fluoride and metal boride. As the protection cover, use may be made of a glass board and a plastic board whose surface is treated so as to reduce water permeability. A method of bonding the cover to a device substrate with a thermosetting resin or a photosetting resin to seal them is suitably used. When a spacer is used to maintain a space, it is easy to protect the device from being damaged. If an inert gas such as nitrogen or argon is injected into the space, oxidation of the cathode can be prevented. Furthermore, if a desiccant such as barium oxide is placed in the space, it is easy to suppress water adsorbed in manufacturing steps from damaging the device. Of these, at least one measure is preferably taken.

A light-emitting device according to the present invention can be used as backlight or illumination for planar light sources, segment display devices, dot matrices and liquid crystal devices.

To obtain a planer light emission using a light-emitting device according to the present invention, a planar anode and a planar cathode may be arranged so as to overlap with each other. Furthermore, to obtain pattern-form light emission, there are a method of providing a mask having a patterned window on the surface of the planar light-emitting device, a method of forming an organic compound layer that corresponds to the portion from which no light is emitted, extremely thick to substantially block light emission, and a method of forming either one or both of an anode and a cathode are formed so as to have a pattern. A pattern is formed by any one of these methods and several electrodes are arranged so as to turn on and off independently. In this manner, a segment-type display device can be obtained which can display numeric characters, letters and simple symbols. Furthermore, to obtain a dot matrix device, stripe form anode and cathode are formed and arranged so as to perpendicularly cross to each other. If a method of separately applying a plurality types of light-emitting materials different in color or a method using a color filter or a light emission conversion filter is employed, partial color display and multicolor display can be attained. The dot matrix device can be passively driven or may be actively driven in combination with TFT and the like. These display devices can be used as display devices of computers, televisions, handheld units, car navigation units and view finders of video cameras.

Furthermore, the planar light-emitting device is a thin film light emitting device and can be suitably used as a planar light source for backlight of liquid crystal devices or a planar illumination light source. Moreover, when a flexible substrate is used, a curved-form light sources and curved-form display devices can be obtained.

Next, as another embodiment of a photoelectric device according to the present invention will be described. As a photoelectric device, for example, a photoelectric conversion device may be mentioned. Examples thereof include a device having a layer containing a metal complex or a composition according to the present invention sandwiched between two pairs of electrodes at least one of which is transparent or translucent, and a device having a comb-form electrode, which is formed on a film-layer containing a polymer compound or a polymer composition according to the present invention and formed on a substrate. To improve characteristics, substances such as fullerene and carbon nanotube may be added.

As a method of producing a photoelectric conversion device, methods described in Japanese Patent No. 3146296 may be exemplified. More specifically, in a method, a polymer thin film is formed on a substrate having a first electrode and a second electrode is formed on the polymer thin film. In another method, a polymer thin film is formed on a pair of comb-form electrodes formed on a substrate. One of the first and second electrodes is transparent or translucent.

A method of forming a polymer thin film and a method of adding fullerene and carbon nanotube are not particularly limited, methods as exemplified with respect to a light-emitting device can be suitably used.

## Examples

The present invention will be explained in detaile below by way of examples. However, the present invention will not be limited to these.

(Synthesis example 1)

Synthesis of compound (L-1)

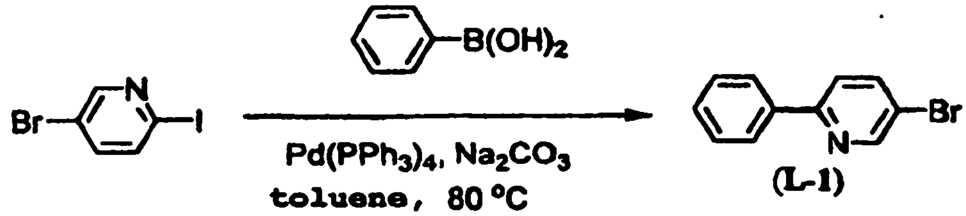

In a reaction container, 2.84g (0.01 mg) of 2-iodo-5-bromopyridine, 1.52g (0.0125 mol) of phenylboric acid and 50 mL of toluene were weighed and placed, and 0.69 g (0.0006 mol) of tetrakis(triphenylphosphine)palladium (0) and a 2M aqueous sodium carbonate solution (10 mL) were added. The reaction solution was stirred with heating under nitrogen airflow at 80°C for 10 hours. The organic layer of the reaction solution was recovered, and then washed with an aqueous sodium carbonate solution (80 mL) and a saturated salt solution (20 mL). The organic layer was dried over sodium sulfate and purified by silica gel column chromatography (hexane/toluene) and the solvent was distilled off to obtain 2.02 g (0.0086 mol) of the compound (L-1) in a yield of 86%. LC-MS (positive) m/z : 234 ([M+H] +)
1 H NMR (300MHz, CDCl3)
δ 7. 4 6 (m, 3H), δ 7.6 3 (d, J = 8. 7Hz, 1H), δ 7. 87 (m, 1H), δ 7. 9 6 (m, 2H), δ 8.74 (s, 1 H) .

(Synthesis example 2)

Synthesis of compound (L-2)

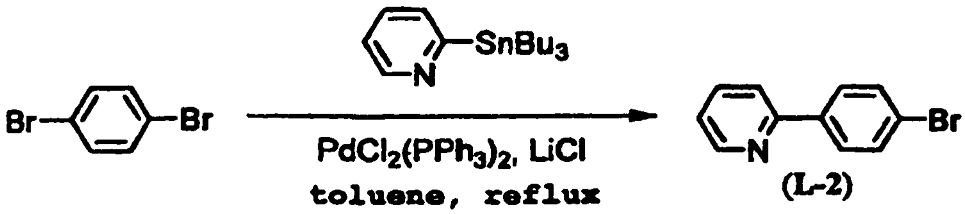


(L-2)

In a reaction container, 29.49 g (0.125 mol) of p-dibromobenzene, 18.78 g (0.05 mol) of tri(n-butyl)(2-pyridyl)tin, 6.36 g (0.15 mol) of lithium chloride and toluene (200 mL) were weighed and placed, and 1.75 g(0.0025 mol) of bis (triphenylphosphine)palladium (II) dichloride was added. The reaction solution was refluxed for 7 hours under nitrogen airflow. To the reaction solution cooled in air, a saturated aqueous potassium fluoride solution (150 mL) was added and the reaction solution was filtrated. After toluene was distilled off, the residue was subjected to extraction with chloroform (500 mL). An extract was washed with a 5% aqueous sodium hydrogen carbonate solution (200 mL). The solvent was distilled off and purification was performed by silica gel column chromatography (hexane/toluene=1/1). The solvent was distilled off to obtain 4.15 g (0.0177 mol) of the compound (L-2) in a yield of 36%.
LC-MS (positive) m/z : 234 ([M+H]+) 1H NMR (300MHz, CDCl3)
δ 7. 2 4 (m,1H), δ 7.6 0 (d, 2H), δ 7.7 2 (m, 2H), δ 7. 88 (d,2H), δ 8. 6 9 (m, 1H).

(Synthesis example 3)

Synthesis of compound (L-3)

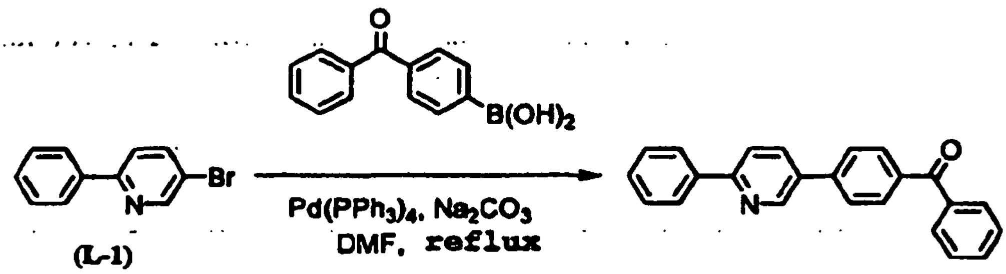


(L-1)

(L-3)

In a reaction container, 1.17 g (0.005 mol) of the compound (L-1), 1.41 g (0.00625 mol) of 4-benzoylphenylboric acid, 1.06 g (0.01 mol) of sodium carbonate, 0.35 g (0.0003 mol) of tetrakis(triphenylphosphine)palladium (0) were weighed and placed, and a dimethylformamide/ethanol solution mixture (35 mL/5 mL) was added. The reaction mixture was refluxed for 7 hours under nitrogen airflow. The reaction mixture was poured in water (100 mL) and extraction was performed with an ethyl acetate/hexane (1/1) solution (100 mL). Thereafter, the organic layer was washed with water (100 mL), a 5% aqueous sodium hydrogen carbonate solution (100 mL) and a saturated salt solution (100 mL). After

the organic layer was dried over sodium sulfate, purification was performed by silica gel column chromatography (toluene/chloroform). The solvent was distilled off to obtain 0.28 g (0.00083 mol) of the compound (L-3) in a yield of 17%.
LC-MS (positive) m/z : 336 ([M+H]+)
1 H NMR (300MHz, CDCl3) δ 7.46 (m, 1H), δ 7.52 (dd, J = 7.5, 7.9 Hz, 4 H), δ 7.61 (m, 1 H), δ 7.77 (d, J = 8.3 Hz, 2 H), δ 7.85 (m, 3 H), δ 7.95 (d, J = 8.1 Hz, 2 H), δ 8.02 (dd, J = 2.4, 8.5 Hz, 1 H), δ 8.07 (m, 2 H), δ 9.00 (s, 1 H).

(Synthesis example 4)

Synthesis of compound (L-4)

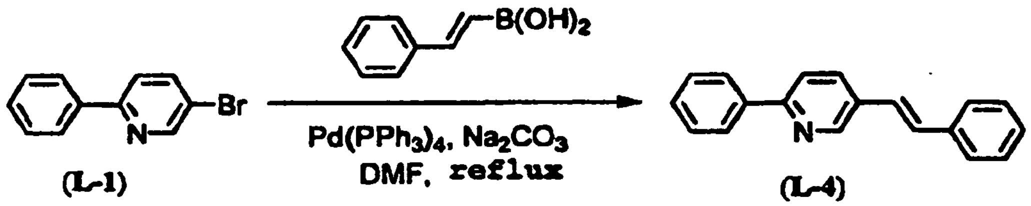


(L-1) (L-4)

In a reaction container, 1.17 g (0.005 mol) of the compound (L-1), 0.76 g (0.00625 mol) of trans-2-phenylvinylboric acid, 1.06g (0.01 mol) of sodium carbonate and 0.35 g (0.0003 mol) of tetrakis (triphenylphosphine)palladium (0) were weighed and placed, and a dimethylformamide/ethanol solution mixture (35 mL/5 mL) was added. The reaction mixture was refluxed for 10 hours under nitrogen airflow. The reaction mixture was poured in water (100 mL) and extraction was performed with an ethyl acetate/hexane (1/1) solution (100 mL). Thereafter, the organic layer was washed with water (100 mL), a 5% aqueous sodium hydrogen carbonate solution (100 mL) and a saturated salt solution (100 mL). After the organic layer was dried over sodium sulfate, purification was performed by silica gel column chromatography (toluene/chloroform). The solvent was distilled off to obtain 0.28 g (0.00083 mol) of the compound (L-4) in a yield of 17%.
L C -MS (positive) m/ z : 2 5 8 ([M+H] +)
1H NMR (300MHz, CDCl3) , δ 7.15 (s, 1H), δ 7.19 (s, 1H), δ 7.31 (d, J = 7.5Hz, 1H), δ 7.39 (m, 3H), δ 7.4 9 (m, 2H), δ 7. 5 6 (d, J = 7.5Hz, 2H), δ 7.75 (d, J = 8.4Hz, 1H), δ 7.9 3 (dd, J = 2.3, 8.3Hz, 1H), δ 8. 03 (d, J = 8.4Hz, 2H), δ 8.79 (s, 1H).

(Synthesis example 5)

Synthesis of compound (L-5)

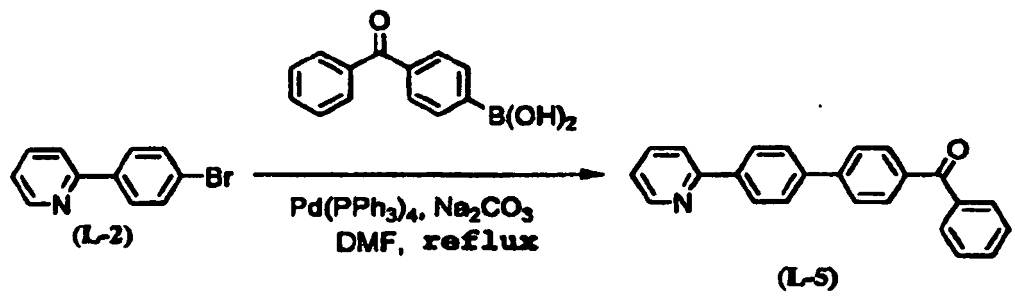


(L-2) (L-5)

In a reaction container, 1.64 g (0.007 mol) of the compound (L-2), 1.98 g (0.00875 mol) of 4-benzoylphenylboric acid, 1.48 g (0.014 mol) of sodium carbonate and 0.49 g (0.00042 mol) of tetrakis(triphenylphosphine)palladium (0) were weighed and placed, and a dimethylformamide/ethanol solution mixture (50 mL/5 mL) was added. The reaction mixture was refluxed for 9 hours under nitrogen airflow. The reaction mixture was poured in water (150 mL) and extraction was performed with an ethyl acetate/hexane (1/1) solution (200 mL). Thereafter, the organic layer was washed with water (150 mL), a 5% aqueous sodium hydrogen carbonate solution (200 mL) and a saturated salt solution (100 mL). After the organic layer was dried over sodium sulfate, purification was performed by silica gel column chromatography (toluene/chloroform). The solvent was distilled off to obtain 0.69 g (0.00206 mol) of the compound (L-5) in a yield of 29%.
LC-MS (positive) m/z : 336 ([M+H]+) 1H NMR (300MHz, CDCl3)

δ 7.2 6 (m,1H), δ 7.51 (m, 2H), δ 7.61 (m, 1H), δ 7. 8 0 (m, 6H), δ 7. 8 6 (d, J = 8.2 H z, 2H), δ 7. 9 2 (d, J=7. 5 H z, 2H), δ 8. 1 3 (d, J = 7.5Hz, 2H), δ 8. 73 (m, 1H).

(Synthesis example 6)

Synthesis of compound (L-6)

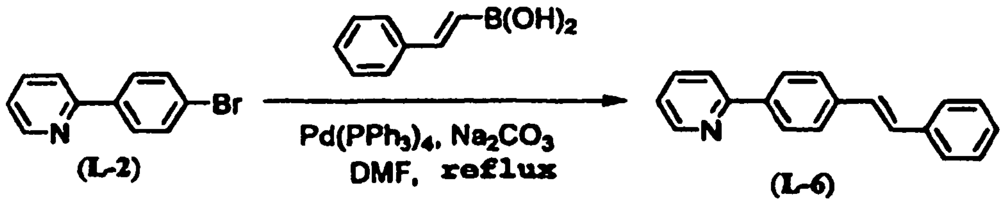


In a reaction container, 1.17g (0.005 mol) of the compound (L-2), 0.76 (0.00625 mol) of trans-2-phenylvinylboric acid, 1.06 g (0.01 mol) of sodium carbonate and 0.35 g (0.0003 mol) of tetrakis (triphenylphosphine)palladium (0) were weighed and placed and a dimethylformamide/ethanol solution mixture (35 mL/5 mL) was added. The reaction mixture was refluxed for 10 hours under nitrogen airflow. The reaction mixture was poured in water (100 mL) and extraction was performed with an ethyl acetate/hexane (1/1) solution (100 mL). The organic layer was washed with water (100 mL), a 5% aqueous sodium hydrogen carbonate solution (100 mL) and a saturated salt solution (100 mL). After the organic layer was dried over sodium sulfate, purification was performed by silica gel column chromatography (toluene/chloroform). The solvent was distilled off to obtain 0.73 g (0.00284 mol) of the compound (L-6) in a yield of 57%.
LC-MS (positive) m/z : 258 ([M+H]+) 1 H NMR (300MHz, CDCl3)
δ 7. 177 (s, 1H), δ 7. 182 (s, 1H), δ 7. 27 (m, 2H), δ 7. 3 8 (dd, J = 7.2, 7.9Hz, 2H), δ 7. 5 5 (d, J = 7.7Hz, 2H), δ 7.63 (d, J = 8. 3Hz, 2H), δ 7. 7 6 (m, 2H), δ 8.0 2 (d, J=8. 3Hz, 2H), δ 8. 70 (d, J=4. 8Hz, 1H).

(Comparative Example 1)

Synthesis of a metal complex (M-3)

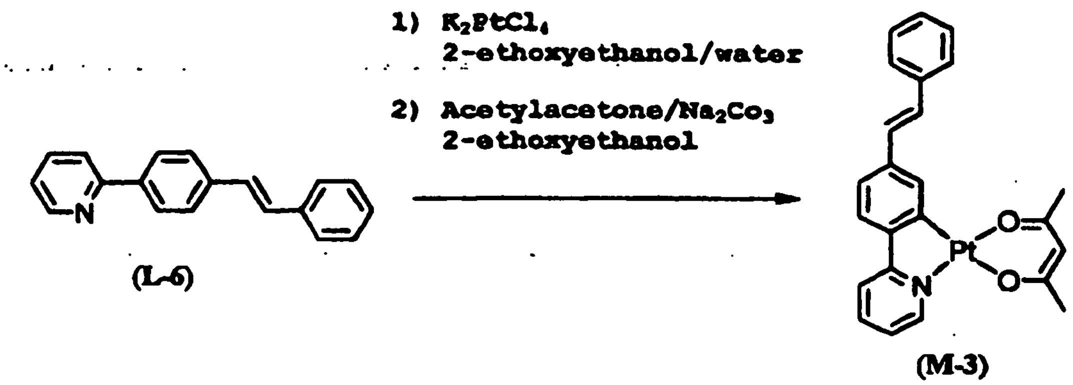


In a reaction container, 591 mg (0.00225 mol) of the compound (L-6), 623 mg (0.0015 mol) of potassium tetrachloroplatinate (II), 180 mL of 2-ethoxyethanol and 60 mL of water were weighed and placed. The reaction mixture was stirred with heating at 80°C for 8 hours. After cooled in air, water (150 mL) was added to the reaction mixture. The reaction product was filtrated and washed with water, methanol and a small amount of methylene chloride successively in this order to obtain a yellow solid substance.
In a reaction container, the yellow solid substance, 207 mg (0.00207 mol) of acetylacetone and 50 mL of 2-ethoxyethanol were weighed and placed. To this, 731 mg (0.0069 mol) of sodium carbonate was added and the reaction mixture was stirred with heating at 100°C for 10 hours. The solvent was distilled off and the residue was dissolved in methylene chloride and purification was performed by silica gel column chromatography (developing solution: methylene chloride).

After concentration, methanol was added to produce a yellow crystal, which was recovered by filtration to obtain 253 mg (0.000460 mol) of the compound (M-3) in a yield of 61%.
LC-MS (positive) m/z : 551 ($[M+H]^+$)
$^1H$ NMR (300 MHz, DMSO-$d_6$)
δ 2.01 (s, 3 H), δ 2.04 (s, 3 H), δ 5.55 (s, 1 H), δ 7.28 (m, 1 H), δ 7.39 (m, 6 H), δ 7.66 (m, 4 H), δ 8.02 (m, 2 H), δ 8.89 (d, J = 5.7 Hz, 1 H).

The metal complex (M-3) and polymethylmethacrylate resin (hereinafter referred to as "PMMA") were mixed in a weight ratio of 2:98 to obtain a 10 wt% toluene solution. Drops of the solution was placed on a quartz substrate, dried to form a PMMA film doped with a metal complex (M-3) on the quartz substrate. PMMA used herein was manufactured by Aldrich.
The substrate prepared above was measured for photoluminescence to obtain quantum efficiency.
The photoluminescence quantum efficiency was measured at an excitation wavelength of 350 nm by use of organic EL luminescence property evaluation apparatus IES-150 manufactured by Optel.

Calculation was performed with respect to the metal complex (M-3), the ratio Φ of the outermost d orbital of the central metal M occupied in the HOMO was as small as 9.9 (%). The difference (ΔE=S1-T1) between the lowest singlet excitation energy (S1) and the lowest triplet excitation energy (T1) was 0.91 (eV) and the oscillator strength f in the lowest singlet excitation was 0.256.

The calculation was performed in accordance with the method described in the detailed description of the invention.
To describe more specifically, using a quantum chemistry calculation program, Gaussian03, and based on the density functional method of the B3LYP level, the structure of a metal complex (M-3) in the ground state was optimized and population analysis of each orbital was performed to obtain the ratio Φ occupied by the outermost d orbital of a central metal in the HOMO. At this time, as the basis function, LANL2DZ was applied to the central metal, whereas 6-31G* to other atoms besides the central metal. Thereafter, the lowest singlet excitation energy (S1) and the lowest triplet excitation energy (T1), were obtained by a time-dependent density functional method of the B3LYP level using the same basis. Then, energy difference (ΔE)=S1-T1 and the oscillator strength f in the lowest singlet excitation was obtained.

(Example 1)

Synthesis of metal complex (M-1)

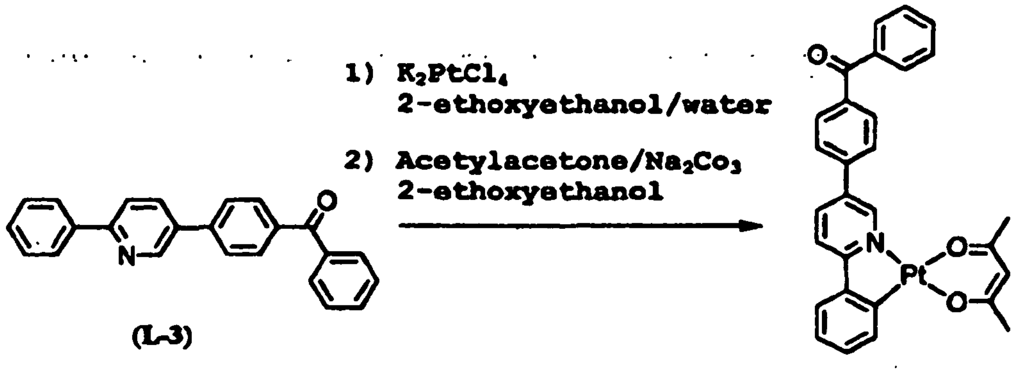


(L-3)

(M-1)

In a reaction container, 268 mg (0.0008 mol) of the compound (L-3), 166 mg (0.0004 mol) of potassium tetrachloroplatinate (II), 45 mL of 2-ethoxyethanol and 15 mL of water were weighed and placed. The reaction mixture was stirred with heating at 80°C for 11 hours. After cooling, water (50 mL) was added. The resultant reaction product was filtrated and washed with water, methanol and a small amount of methylene chloride to obtain a yellow solid substance.
In a reaction container, the yellow solid substance, 60 mg (0.0006 mol) of acetylacetone and 15 mL of 2-ethoxyethanol were weighed and placed. To this, 212 mg (0.002 mol) of sodium carbonate was added. The reaction mixture was stirred with heating at 100°C for 5 hours. The solvent was distilled off and the residue was dissolved in methylene chloride and purification was performed by silica gel column chromatography (developing solution: methylene chloride). After concentrated, methanol was added to produce a yellow crystal, which was recovered by filtration to obtain 72 mg (0.000114 mol) of the compound (M-1) in a yield of 28%.
LC-MS (positive) m/z : 629 ([M+H]’)

$^1H$ NMR (300 MHz, $DMSO-d_6$)
δ 1.96 (s, 3 H), δ 2.01 (s, 3 H), δ 5.60 (s, 1 H), δ 7.11 (m, 2 H), δ 7.43 (d, J = 7.3 Hz, 1 H), δ 7.59 (t, J = 7.4 Hz, 2 H), δ 7.71 (m, 2 H), δ 7.79 (d, J = 7.5 Hz, 2 H), δ 7.93 (m, 4 H), δ 8.12 (d, J = 8.6 Hz, 1 H), δ 8.46 (d, J = 7.4 Hz, 1 H), 5 9.22 (s, 1 H).

A PMMA film doped with the metal complex (M-1) was formed using the metal complex (M-1) in the same manner as in Comparative Example 1 and photoluminescence was measured. Light emission having peaks at 545 nm and 583 nm was observed. The quantum efficiency was 72.9 fold as high as that in Comparative Example 1.

0.8 wt% of a chloroform solution containing a mixture, in which the following compound (CBP) and the metal complex (M-1) were contained in a weight ratio of 97.5:2.5, was prepared and an EL device was manufactured. Note that CBP was purchased from Dojindo Laboratories.

(CBP)

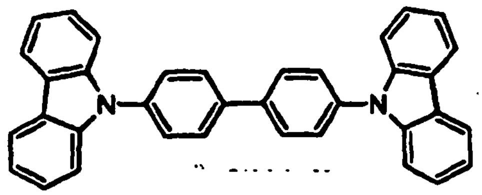

To a glass substrate on which an ITO film of 150 nm thick was previously formed by a sputtering method, a solution of poly(ethylenedioxythiophene)/polystyrene sulfonate (BaytronP manufactured by Bayer) was applied by spin coating to form a film of 50 nm thick, which was dried on a hot plate at 200°C for 10 minutes. Subsequently, the chloroform solution prepared above was applied to form a film by spin coating at a rotation speed of 2500 rpm. The film was dried under a nitrogen gas atmosphere at 130°C for one hour. Thereafter, barium was deposited to a thickness of about 5 nm as a cathode, and then, aluminum was deposited to a thickness of about 80 nm to obtain an EL device. Note that after the degree of vacuum reached to $1 \times 10^{-4}$Pa or less, deposition of a metal was initiated.
When voltage was applied to the device thus obtained, light emission having a maximum peak at 540 nm was observed. The device provided light emission having a brightness as high as about 100 cd/$m^2$ at 18.5V.

Calculation was performed with respect to the metal complex (M-1) in the same manner as in Comparative Example 1. As a result, the ratio Φ occupied by the outermost d orbital of the central metal M in the HOMO was 41.4 (%). The energy difference (ΔE) between the lowest singlet excitation energy (S1) and the lowest triplet excitation energy (T1), that is, ΔE =S1-T1, was 0.42 (eV), and the oscillator strength f in the lowest singlet excitation was 0.122.

(Reference Example 2)

Synthesis of metal complex (M-2)

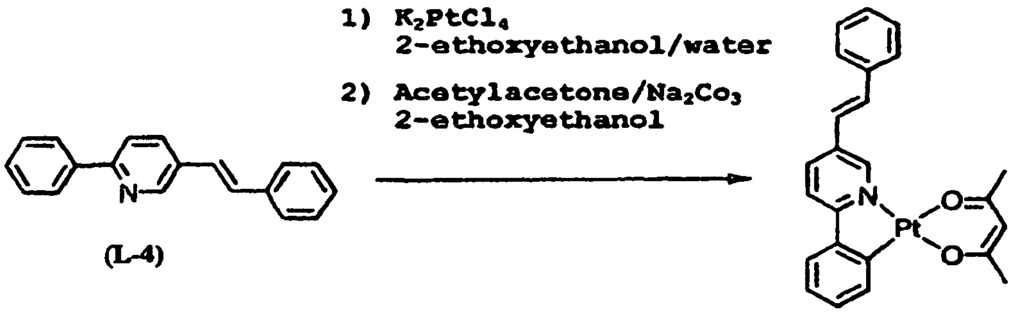


(L-4)

(M-2)

In a reaction container, 412 mg (0.0016 mol) of the compound (L-4), 332 mg (0.0008 mol) of potassium tetrachloroplatinate (II), 90 mL of 2-ethoxyethanol and 30 mL of water were weighed and placed. The reaction mixture was stirred with heating at 80°C for 10 hours. After cooling, water (100 mL) was added. The resultant reaction product was filtrated and

washed with water, methanol and a small amount of methylene chloride to obtain a yellow solid substance.
In a reaction container, the yellow solid substance, 120. mg (0.0012 mol) of acetylacetone and 30 mL of 2-ethoxyethanol were weighed and placed. To this, 424 mg (0.004 mol) of sodium carbonate was added, and the reaction mixture was stirred with heating at 100°C for 9 hours. The solvent was distilled off and the residue was dissolved in methylene chloride and purification was performed by silica gel column chromatography (developing solution: methylene chloride). After concentrated, methanol was added to produce a yellow crystal, which was recovered by filtration to obtain 62 mg (0.000113 mol) of the compound (M-2) in a yield of 14%.
LC-MS (positive) m/z : 551 ($[M+H]^+$)
$^1H$ NMR (300 MHz, DMSO-$d_6$)
δ 1.97 (s, 3 H), δ 2.07 (s, 3 H), δ 5.61 (s, 1 H), δ 7.10 (m, 2 H), δ 7.34 (m, 1 H), δ 7.43 (m, 5 H), δ 7.68 (m, 3 H), δ 8.01 (d, J = 8.6 Hz, 1 H), δ 8.39 (d, J = 8.6 Hz, 1 H), δ 8.98 (s, 1 H).

A PMMA film doped with the metal complex (M-2) was formed using the metal complex (M-2) in the same manner as in Example 1 and photoluminescence was measured. The quantum efficiency was 1.3 fold as high as that in Comparative Example 1.

Calculation was performed with respect to the metal complex (M-2) in the same manner as in Example 1. As a result, the ratio Φ occupied by the outermost d orbital of the central metal M in the HOMO was 34.7 (%). The energy difference (ΔE) between the lowest singlet excitation energy (S1) and the lowest triplet excitation energy (T1), that is, ΔE =S1-T1, was 0.74 (eV), and the oscillator strength f in the lowest singlet excitation was 0.223.

(Example 3)

Synthesis of metal complex (M-4)

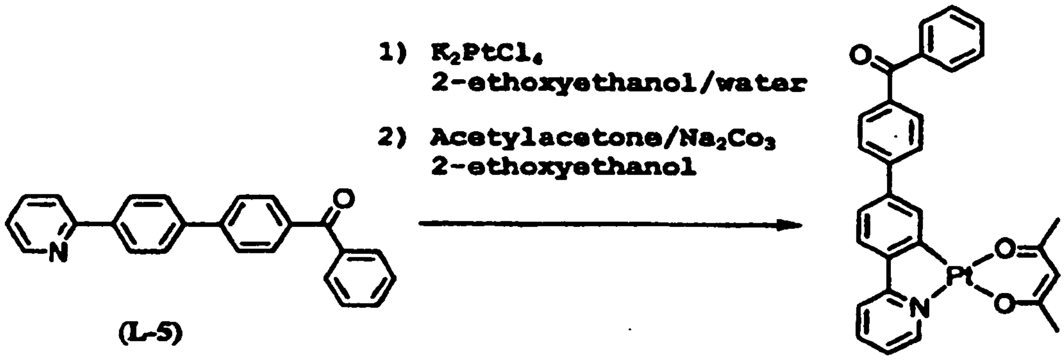


(L-5)

(M-4)

In a reaction container, 587 mg (0.00175 mol) of the compound (L-4), 581 mg (0.0014 mol) of potassium tetrachloroplatinate (II), 120 mL of 2-ethoxyethanol and 40 mL of water were weighed and placed. The reaction mixture was stirred with heating at 80°C for 10 hours. After cooling, water (200 mL) was added. The resultant reaction product was filtrated and washed with water, methanol and a small amount of methylene chloride to obtain a yellow solid substance.
In a reaction container, the yellow solid substance, 120 mg (0.0012 mol) of acetylacetone and 30 mL of 2-ethoxyethanol were weighed and placed. To this, 424 mg (0.004 mol) of sodium carbonate was added, and the reaction mixture was stirred with heating at 100°C for 10 hours. The solvent was distilled off and the residue was dissolved in methylene chloride and purification was performed by silica gel column chromatography (developing solution: methylene chloride). After concentrated, methanol was added to produce a yellow crystal, which was recovered by filtration to obtain 145 mg (0.000213 mol) of the compound (M-4) in a yield of 29%.
LC-MS (positive) m/z : 629 ($[M+H]^+$)
$^1H$ NMR (300 MHz, DMSO-$d_6$)
δ 2.00 (s, 3 H), δ 2.02 (s, 3 H), δ 5.62 (s, 1 H), δ 7.46 (m, 2 H), δ 7.60 (m, 2 H), δ 7.70 (m, 2 H), δ 7.80 (m, 3 H), δ 7.88 (m, 4 H), δ 8.08 (m, 2 H), δ 8.93 (d, J = 6.1 Hz, 1 H).

A PMMA film doped with the metal complex (M-4) was formed using the metal complex (M-4) in the same manner as in Example 1 and photoluminescence was measured. Light emission having peaks at 544 nm and 581 nm was observed. The quantum efficiency was 48.6 fold as high as that in Comparative Example 1.

Calculation was performed with respect to the metal complex (M-4) in the same manner as in Example 1. As a result, the ratio Φ occupied by the outermost d orbital of the central metal M in the HOMO was 39.1 (%). The energy

difference (ΔE) between the lowest singlet excitation energy (S1) and the lowest triplet excitation energy (T1), that is, ΔE =S1-T1, was 0.56 (eV), and the oscillator strength f in the lowest singlet excitation was 0.093.

(Example 4)

Calculation was performed with respect to the metal complexes (M-5) and (M-6) in the same manner as in the case of the metal complex (M-1). As a result, the ratios Φ occupied by the outermost d orbital of the central metal M in the HOMO, the energy differences (ΔE) between the lowest singlet excitation energy (S1) and the lowest triplet excitation energy (T1), that is, ΔE =S1-T1, and the oscillator strength f values in the lowest singlet excitation were obtained as shown in Table 1.
The metal complex (M-6) is superior to the metal complex (M-5) of a comparative example, since the values of Φ and f are larger.

[Table 1]

| | Φ (%) | ΔE (eV) | f |
|---|---|---|---|
| M-5 | 0.0 | 0.65 | 0.007 |
| M-6 | 48.4 | 0.24 | 0.029 |

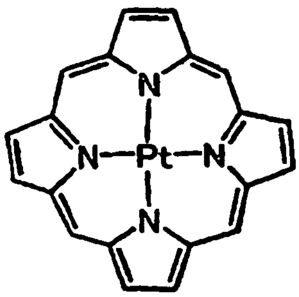

(M-5)

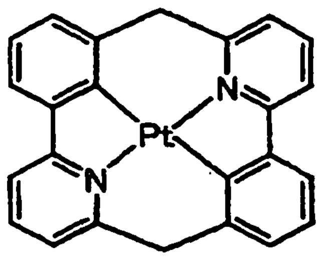

(M-6)

(Reference Example 5)

Calculation was performed with respect to the following metal complex (M-7) in the same manner as in the case of the metal complex (M-1). As a result, the ratio Φ occupied by the outermost d orbital of the central metal M in the HOMO was 37.2%. The energy difference (ΔE) between the lowest singlet excitation energy (S1) and the lowest triplet excitation energy (T1), that is, ΔE =S1-T1, was 0.24 (eV). The oscillator strength f in the lowest singlet excitation was 0.033.

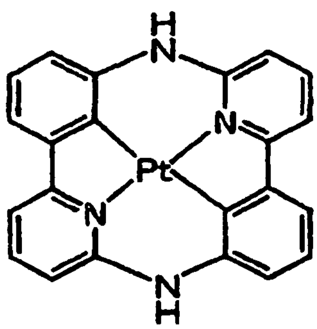

(M-7)

(Reference Example 6)

Calculation was performed with respect to the following metal complex (M-8) in the same manner as in the

case of the metal complex (M-1). As a result, the ratio Φ occupied by the outermost d orbital of the central metal M in the HOMO was 45.7%. The energy difference (AE) between the lowest singlet excitation energy (S1) and the lowest triplet excitation energy (T1), that is, ΔE =S1-T1, was 0.22 (eV). The oscillator strength f in the lowest singlet excitation was 0.026.

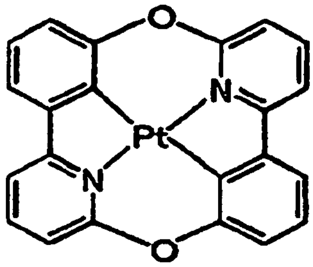

(M-8)

(Reference Example 7)

Calculation was performed with respect to the following metal complex (M-9) in the same manner as in the case of the metal complex (M-1). As a result, the ratio Φ occupied by the outermost d orbital of the central metal M in the HOMO was 36.6%. The energy difference (ΔE) between the lowest singlet excitation energy (S1) and the lowest triplet excitation energy (T1), that is, ΔE =S1-T1, was 0.24 (eV). The oscillator strength f in the lowest singlet excitation was 0.038.

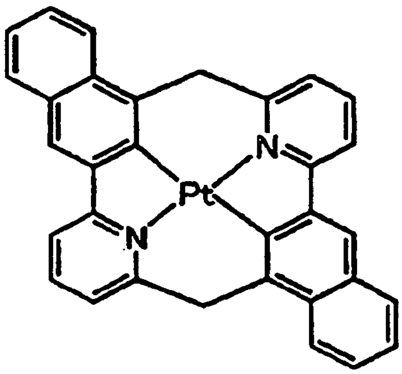

(M-9)

(Reference Example 8)

Calculation was performed with respect to the following metal complex (M-10) in the same manner as in the case of the metal complex (M-1). As a result, the ratio Φ occupied by the outermost d orbital of the central metal M in the HOMO was 41.8%. The energy difference (ΔE) between the lowest singlet excitation energy (S1) and the lowest triplet excitation energy (T1), that is, ΔE =S1-T1, was 0.24 (eV). The oscillator strength f in the lowest singlet excitation was 0.023.

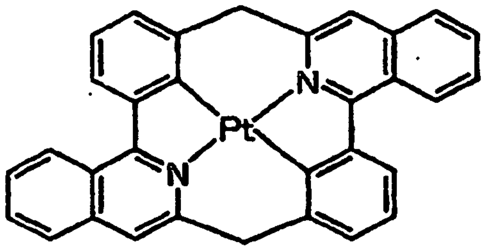

(M−10)

INDUSTRIAL APPLICABILITY

A metal complex according to the present invention is a complex using a metal having a larger production and reserve than iridium as the central metal, and excellent in luminous efficiency, stability and color purity, etc. Therefore, use of the metal complex of the present invention in a photoelectric device such as electroluminescent device can render the characteristics of the device to be more excellent.

**Claims**

1. A metal complex **characterized by** having a partial structure represented by the general formula (1-1) below:

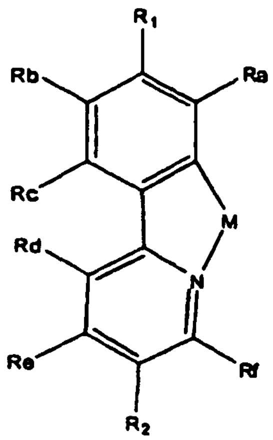

(1-1)

wherein M represents the central metal; $R_1$ and $R_2$ and Ra to Rf each independently represent a hydrogen atom, an alkyl group, an alkoxy group, a phenyl group or a halogen atom, provided that at least one of $R_1$ and $R_2$ is a group represented by the formula (2) below,

wherein $R_{11}$ to $R_{19}$ each independently represent a hydrogen atom, an alkyl group, an alkoxy group, a phenyl group or a halogen atom, and the wave line indicates a binding position.

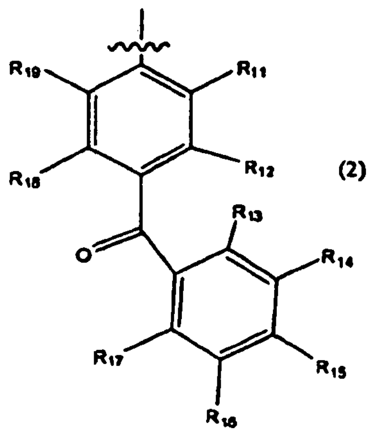

(2)

2. A metal complex according to claim 1 having a central metal M selected from the group consisting of Sc, Ti, V, Cr, Mn, Fe, Co, Ni, Cu, Zn, Y, Zr, Nb, Mo, Tc, Ru, Rh, Pd, Ag, Cd, Hf, Ta, W, Re, Pt, Au and Hg, wherein no halogen unidentate ligand is contained in the complex; and a ratio of a sum of squares of orbital coefficients of outermost d orbitals of the central metal relative to a sum of squares of all atomic orbital coefficients in a highest occupied molecular orbital (HOMO) obtained by a computational scientific technique is 1/3 or more; an energy difference (S1-T1) between a lowest singlet excitation energy (S1) and a lowest triplet excitation energy (T1) obtained by a computational scientific technique falls within 0.1 (eV) to 1.0 (eV) (both inclusive); and an oscillator strength (f) in the lowest singlet excited state falls within a range of 0.005 to 1.0 (both inclusive).

3. The metal complex according to claim 2, wherein the oscillator strength (f) and the energy difference (S1-T1) are defined by the equations:

$$f \leq 0.24 \times (S1-T1) + 0.06$$

$$f \geq 0.24 \times (S1-T1) - 0.06$$

4. The metal complex according to claim 3, wherein the energy difference (S1-T1) falls within a range of 0.1 (eV) to 0.28 (eV) (both inclusive) and the oscillator strength (f) is 0.005 or more.

5. The metal complex according to claim 3, wherein the energy difference (S1-T1) is larger than 0.28 (eV) and 1 (ev) or less and the oscillator strength (f) is 0.035 or more.

6. The metal complex according to any one of claims 2 to 4, wherein the energy difference (S1-T1) falls within a range of 0.15 (eV) to 0.28 (eV) (both inclusive) and the oscillator strength (f) falls within the range of 0.025 to 0.05 (both inclusive).

7. The metal complex according to claim 3 or 5,
wherein the energy difference (S1-T1) is larger than 0.28 (eV) and 1.0 (eV) or less and the oscillator strength (f) falls within a range of 0.07 to 0.3 (both inclusive).

8. The metal complex according to claim 3 or 5,
wherein the energy difference (S1-T1) is larger than 0.28 (eV) and 1.0 (eV) or less and the oscillator strength (f) falls within a range of 0.09 to 0.3 (both inclusive).

9. The metal complex according to claim 3 or 5,
wherein the energy difference (S1-T1) is larger than 0.28 (eV) and 1.0 (eV) or less and the oscillator strength (f) falls within a range of 0.10 to 0.25 (both inclusive).

10. The metal complex according to any one of claims 2 to 9, being nonionic.

11. The metal complex according to any one of claims 2 to 10, having at least one multidentate chelate ligand containing at least one aromatic ring.

12. A composition **characterized by** comprising the metal complex according to any one of claims 1 to 11 and a charge transport material.

13. The composition according to claim 12, wherein the charge transport material is an organic compound.

14. The composition according to claim 13, wherein the organic compound is a low molecular weight organic compound.

15. The composition according to claim 13, wherein the organic compound is a polymer.

16. The composition according to claim 15, wherein the polymer is a conjugated polymer.

17. The composition according to claim 16, wherein the polymer contains repeat units represented by the general formula (6) below,

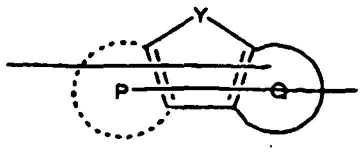

(6)

wherein P ring and Q ring each independently represent an aromatic ring; however, the P ring may not be present; two bonds are present on the P ring and/or the Q ring when the P ring is present, and present on the 5-membered ring or 6-membered ring each containing Y and/or on the Q ring when the P ring is not present; a substituent may be present on the aromatic ring and/or the 5-membered ring or 6-membered ring containing Y; Y represents -O-, -S-, -Se-, -B($R_{31}$)-, -N($R_{35}$)-, -C(R1)(R2)-, -Si($R_1$) ($R_2$)-, -P($R_3$)-, -P$R_4$(=O) -, -C($R_{51}$) ($R_{52}$) -C($R_{53}$) ($R_{54}$) -O-C ($R_{55}$) ($R_{56}$)-, -S-C($R_{57}$) ($R_{58}$)-, -N-C($R_{59}$) ($R_{60}$)-, -Si ($R_{61}$) ($R_{62}$)-C($R_{63}$) ($R_{64}$)-, -Si ($R_{65}$) ($R_{66}$) -Si ($R_{67}$) ($R_{68}$)-, -C($R_{69}$) =C ($R_{70}$)-, -N=C($R_{71}$) - or -Si($R_{72}$)=C($R_{73}$)-; $R_1$ to $R_4$, $R_{31}$, $R_{35}$ and $R_{51}$ to $R_{73}$ each independently represent a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a silyloxy group, a substituted silyloxy group, a monovalent heterocyclic group or a halogen atom.

18. A polymer **characterized by** containing the metal complex according to any one of claims 1 to 11 as a partial structure in a molecule.

19. The polymer according to claim 18 being a conjugated polymer.

20. A composition **characterized by** containing at least one type of material selected from the group consisting of the composition or the polymer according to any one of claims 12 to 19, a hole transport material, an electron transport material and a light-emitting material.

21. An ink composition **characterized by** containing the metal complex, composition or polymer according to claims 1 to 20.

22. The ink composition according to claim 21, having a viscosity of 1 to 100 mPa·s at 25°C.

23. A light emitting thin film **characterized by** containing the metal complex, composition or polymer according to claims 1 to 20.

24. A photoelectric device **characterized by** containing the metal complex, composition or polymer according to claims 1 to 20.

25. A photoelectric device **characterized by** comprising a layer containing the composition or polymer according to any one of claims 12 to 20 between electrodes consisting of an anode and a cathode.

26. The photoelectric device according to claim 25, further comprising a charge transport layer or a charge blocking layer between the electrodes consisting of an anode and a cathode.

27. The photoelectric device according to any one of claims 24 to 26, wherein the photoelectric device is a light-emitting device.

28. A planar light source **characterized by** using the light-emitting device according to claim 27,

29. A segment display device **characterized by** using the light-emitting device according to claim 27.

30. A dot matrix display device **characterized by** using the light-emitting device according to claim 27.

31. A liquid crystal display device **characterized by** using the light-emitting device according to claim 27 as backlight.

32. Illumination **characterized by** using the light-emitting device according to claim 27.

## Patentansprüche

1. Ein Metallkomplex, **dadurch gekennzeichnet, dass** er eine Teilstruktur aufweist, die von der nachstehenden allgemeinen Formel (1-1) dargestellt wird:

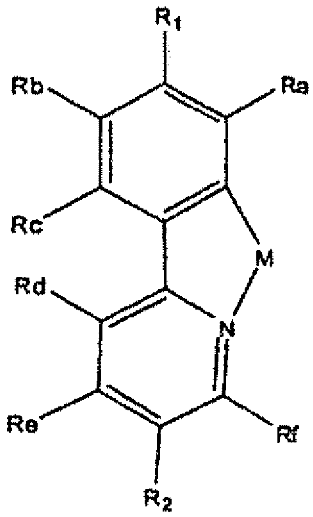

(1-1)

wobei M das zentrale Metall darstellt, $R_1$ und $R_2$ und Ra bis Rf jeweils unabhängig ein Wasserstoffatom, einen Alkylrest, einen Alkoxyrest, einen Phenylrest oder ein Halogenatom darstellen, mit der Maßgabe, dass mindestens einer von $R_1$ und $R_2$ ein Rest ist, der von der untenstehenden Formel (2) dargestellt wird,
wobei $R_{11}$ bis $R_{19}$ jeweils unabhängig ein Wasserstoffatom, einen Alkylrest, einen Alkoxyrest, einen Phenylrest oder ein Halogenatom darstellen und die geringelte Linie eine Bindeposition angibt.

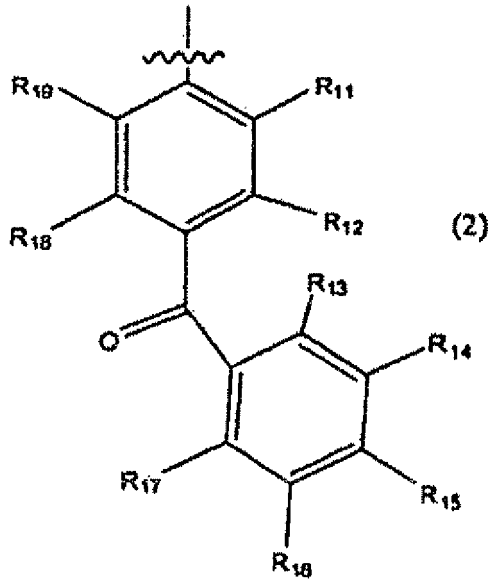

(2)

2. Ein Metallkomplex gemäß Anspruch 1, der ein zentrales Metal M aufweist, das ausgewählt ist aus der Gruppe bestehend aus Sc, Ti, V, Cr, Mn, Fe, Co, Ni., Cu, Zn, Y, Zr, Nb, Mo, Tc, Ru, Rh, Pd, Ag, Cd, Hf, Ta, W, Re, Pt, Au und Hg, wobei kein einzähniger Halogenligand in dem Komplex enthalten ist;
und ein Verhältnis einer Summe von Quadraten von Orbitalkoeffizienten von äußersten d-Orbitalen des zentralen Metalls zu einer Summe von Quadraten von allen Atom-Orbitalkoeffizienten in einem höchsten besetzten Molekül-Orbital (HOMO - Highest Occupied Molecular Orbital), das durch eine rechnerische wissenschaftliche Methode erhalten wird, 1/3 oder mehr beträgt; eine Energiedifferenz (S1-T1) zwischen einer niedrigsten Singlett-Anregungsenergie (S1) und einer niedrigsten Triplett-Anregungsenergie (T1), erhalten durch eine rechnerische wissenschaftliche Methode, zwischen 0,1 (eV) und 1,0 (eV) (beide inklusive) liegt; und eine Oszillatorstärke (f) im niedrigsten Singlett-Anregungszustand innerhalb eines Bereiches von 0,005 bis 1,0 (beide inklusive) liegt.

3. Der Metallkomplex gemäß Anspruch 2, wobei die Oszillatorstärke (f) und die Energiedifferenz (S1-T1) definiert sind durch die Gleichungen:

$$f \leq 0{,}24 \times (S1\text{-}T1) + 0{,}06$$

$$f \geq 0{,}24 \times (S1\text{-}T1) - 0{,}06$$

4. Der Metallkomplex gemäß Anspruch 3, wobei die Energiedifferenz (S1-T1) innerhalb eines Bereichs von 0,1 (eV) bis 0,28 (eV) (beide inklusive) liegt und die Oszillatorstärke (f) 0,005 oder mehr beiträgt.

5. Der Metallkomplex gemäß Anspruch 3, wobei die Energiedifferenz (S1-T1) größer als 0,28 (eV) und 1 (eV) oder kleiner ist und die Oszillatorstärke (f) 0,035 oder mehr beträgt.

6. Der Metallkomplex gemäß einem der Ansprüche 2 bis 4, wobei die Energiedifferenz (S1-T1) innerhalb eines Bereichs von 0,15 (eV) bis 0,28 (eV) (beide inlclusive) liegt und die Oszillatorstärke (f) innerhalb des Bereichs von 0,025 bis 0,05 (beide inklusive) liegt.

7. Der Metallkomplex gemäß Anspruch 3 oder 5, wobei die Energiedifferenz (S1-T1) größer als 0,28 (eV) und 1,0 (eV) oder kleiner ist und die Oszillatorstärke (f) innerhalb eines Bereichs von 0,07 bis 0,3 (beide inlclusive) liegt.

8. Der Metallkomplex gemäß Anspruch 3 order 5, wobei die Energiedifferenz (S1-T1) größer als 0,28 (eV) und 1,0 (eV) oder kleiner ist und die Oszilatorstärke (f) innerhalb eines Bereichs von 0,09 bis 0,3 (beide inklusive) liegt.

9. Der Metallkomplex gemäß Anspruch 3 oder 5, wobei die Energiedifferenz (S1-T1) größer als 0,28 (eV) und 1,0 (eV)

oder kleiner ist und die Oszillatorstärke (f) innerhalb eines Bereichs von zu bis 0,25 (beide inlclusive) liegt.

**10.** Der Metallkomplex gemäß einem der Ansprüche 2 bis 9, wobei der Metallkomplex nichtionisch ist.

**11.** Der Metallkomplex gemäß einem der Ansprüche 2 bis 10, wobei der Metallkomplex mindestens einen mehrzähnigen Chelatliganden aufweist, der mindestens einen aromatischen Ring enthält.

**12.** Eine Zusammensetzung, **dadurch gekennzeichnet, dass** sie den Metallkomplex gemäß einem der Ansprüche 1 bis 11 und ein Ladung transportiereaades Material umfasst.

**13.** Die Zusammensetzung gemäß Anspruch 12, wobei das Ladung transportierende Material eine organische Verbindung ist.

**14.** Die Zusammensetzung gemäß Anspruch 13, wobei die organische Verbindung eine orgazaische Verbindung mit geringem Molekulargewicht ist.

**15.** Die Zusammensetzung gemäß Anspruch 13, wobei die organische Verbindung ein Polymer ist.

**16.** Die Zusammensetzung gemäß Anspruch 15, wobei das Polymer ein konjugiertes Polymer ist.

**17.** Die Zusammensetzung gemäß Anspruch 16, wobei das Polymer Wiederholungseinheiten enthält, die von der untenstehenden allgemeinen Formel (6) dargstellt werden,

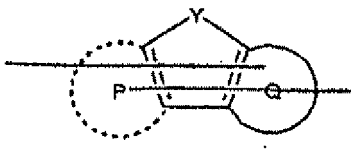

(6)

wobei der P-Ring und der Q-Ring jeweils unabhängig einen aromatische Ring darstellen; der P-Ring jedoch gegebenenfalls nicht vorhanden ist, zwei Bindungen am P-Ring und/oder Q-Ring vorhanden sind, wenn der P-Ring, vorhanden ist, und am 5-gliedrigen Ring oder 6-gliedrigen Ring, wobei jeder Y enthält, vorhanden sind und/oder am Q-Ring vorhanden sind, wenn der P-Ring, nicht vorhanden ist; ein Substituent an dem aromatischen Ring und/oder dem 5-gliedrigen Ring oder 6-gliedrigen Ring, der Y enthält, vorhanden sein kann; Y -O-, -S-, -Se-, -B($R_{31}$)-, -N($R_{35}$)-, -C($R_1$)($R_2$)-, -Si($R_1$)($R_2$)-, -P($R_3$)-,- P$R_4$(=O)-, -C($R_{51}$)($R_{52}$)-C($R_{53}$)($R_{54}$)-, -O-C($R_{55}$)($R_{56}$)-, -S-C($R_{57}$)($R_{58}$)-, -N-C($R_{59}$)($R_{60}$)-, -Si($R_{61}$)($R_{62}$)-C($R_{63}$)($R_{64}$)-, Si($R_{65}$)($R_{66}$)-Si($R_{67}$)($R_{68}$)-, -C($R_{69}$)=C($R_{70}$)-, -N=C($R_{71}$)- oder -Si($R_{12}$) =C($R_{73}$)- darstellt; $R_1$ bis $R_4$, $R_{31}$, $R_{35}$ und $R_{51}$ bis $R_{73}$ jeweils unabhängig ein Wasserstoffatom, einen Alkylrest, einen Alkoxyrest einen Alkyllthiorest, einen Arylrest, einen Aryloxyrest, einen Azylthiorest, einen Arylalkylrest, einen Arylakoxyrest,
einen Arylalkylthiarcst, einen Arylalkenylrest, einen Arylalkinylrest, eine Aminogruppe, eine substituierte Aminogruppe, eine Silylgruppe, eine substituierte Silylgruppe, eine Siloxygruppe, eine substituierte Silyloxygruppe, eine monovalente heterocyclische Gruppe oder ein Halogenatom darstellen.

**18.** Ein Polymer, **dadurch gekennzeichnet, dass** es den Metallkomplex gemäß einem der Anspruche 1 bis 11 als eine Teilstruktur in einem Molekül enthält.

**19.** Das Polymer gemäß Anspruch 18, wobei es sich um ein konjugiertes Polymer handelt.

**20.** Eine Zusammensetzung, **dadurch gekennzeichnet, dass** sie mindestens eine Art von Material, ausgewählt aus der Gruppe bestehend aus der Zusammensetzung oder dem Polymer gemäß einem der Ansprüche 12 bis 19, ein Lochtransportmaterial, ein Elektrontransportmaterial und ein Licht emittierendes Material, enthält.

**21.** Eine Tintenzusammensetzung, **dadurch gekennzeichnet, dass** sie den Metallkomplex, die Zusammensetzung oder das Polymer gemäß den Ansprüche 1 bis 20 enthält.

**22.** Die Tintenzusammensetzung gemäß Anspruch 21, die eine Viskosität von 1 bis 100 mPa·s bei 25°C aufweist.

**23.** Eine Licht emittierende Dünnschicht, **dadurch gekennzeichnet, dass** sie den Metallkomplex, die Zusammensetzung oder das Polymer gemäß den Ansprüchen 1 bis 20 enthält.

**24.** Ein photoelektrisches Gerät, **dadurch gekennzeichnet, dass** es den Metallkomplex, die Zusammensetzung oder das Polymer gemäß den Ansprüchen 1 bis 20 enthält.

**25.** Ein photoelektrisches Gerät, **dadurch gekennzeichnet, dass** es eine Schicht umfasst, die die Zusammensetzung oder das Polymer gemäß einem der Ansprüche 12 bis 20 zwischen Elektroden, bestehend aus einer Anode und einer Kathode, enthält.

**26.** Das photoelektrische Gerät gemäß Anspruch 25, weiter umfassend eine Ladung transportierende Schicht oder eine Ladung blockierend Schicht zwischen den Elektroden, bestehend aus einer Anode und einer Kathode.

**27.** Das photoelektrische Gerät gemäß einem der Ansprüche 24 bis 26, wobei das photoelektrische Gerät ein Licht emittierendes Gerät ist.

**28.** Eine flache Lichtquelle, **dadurch gekennzeichnet, dass** darin das Licht emittierende Gerät gemäß Anspruch 27 verwendet wird.

**29.** Eine Segmentanzeigevorrichtung, **dadurch gekennzeichnet, dass** darin das Licht emittierende Gerät gemäß Anspruch 27 verwendet wird.

**30.** Eine Dot-Matrix-Anzeigevorrichtung, **dadurch gekennzeichnet, dass** darin das Licht emittierende Gerät gemäß Anspruch 27 verwendet wird.

**31.** Eine Flüssigkristallanzeigevorrichtung, **dadurch gekennzeichnet, dass** darin das Licht emittierende Gerät gemäß Anspruch 27 als Hintergrundbeleuchtung verwendet wird.

**32.** Beleuchtung, **dadurch gekennzeichnet, dass** darin das Licht emittierende Gerät gemäß Anspruch 27 verwendet wird.

## Revendications

**1.** Complexe de métal **caractérisé en ce qu'**il a une structure partielle représentée par la formule générale (1-1) ci-dessous :

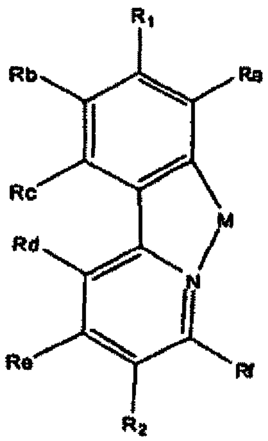

(1-1)

dans lequel M représente le métal central ; $R_1$ et $R_2$ et Ra à Rf représentent chacun indépendamment un atome d'hydrogêne, un groupe alkyl, un groupe alcoxy, un groupe phényle ou un atome d'halogène, à condition qu'au moins l'un de $R_1$ et $R_2$ soit un groupe représenté par la formule (2) ci-dessous,
dans lequel $R_{11}$ à $R_{19}$ représentent chacun indépendamment un atome d'hydrogène, un groupe alkyle, un groupe alcoxy, un groupe phényle ou un atome d'halogène, et la ligne ondulée indique une position de liaison.

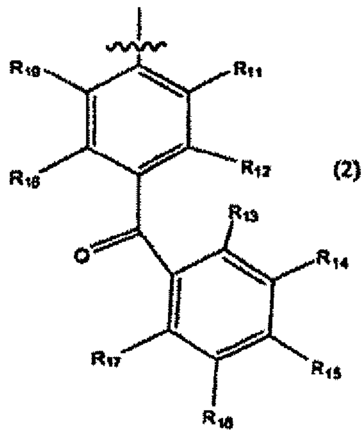

(2)

.

**2.** Complexe de métal selon la revendication 1, ayant un métal central M choisi dans le groupe constitué par Sc, Ti, V, Cr, Mn, Fe, Co, Ni, Cu, Zn, Y, Zr, Nb, Mo, Tc, Ru, Rh, Pd, Ag, Cd, Hf, Ta, W, Re, Pt, Au et Hg, dans lequel aucun ligand monodenté halogène n'est contenu dans le complexe ; et un rapport entre une somme des carrés des coefficients orbitalaires des orbitales d les plus externes du métal central et une somme des carrés de tous les coefficients orbitalaires atomiques dans une orbitale moléculaire occupée la plus haute (HOMO) obtenu par une technique scientifique de calcul est de 1/3 ou plus ; une différence d'énergie (S1-T1) entre une énergie d'excitation singulet la plus faible (S1) et une énergie d'excitation triplet la plus faible (T1) obtenue par une technique scientifique de calcul se situe entre 0,1 (eV) à 1,0 (eV) (les deux valeurs étant incluses) ; et une intensité d'oscillateur (f) dans l'état excité singulet le plus faible se situe dans une plage de 0,005 à 1,0 (les deux valeurs étant incluses).

**3.** Complexe de métal selon la revendication 2, dans lequel l'intensité d'oscillateur (f) et la différence d'énergie (S1 - T1) sont définies par les équations :

$$f \leq 0,24 \times (S1 - T1) + 0,06$$

$$f \geq 0,24 \times (S1 - T1) - 0,06.$$

**4.** Complexe de métal selon la revendication 3, dans lequel la différence d'énergie (S1 - T1) se situe dans une plage de 0,1 (eV) à 0,28 (eV) (les deux valeurs étant incluses) et l'intensité d'oscillateur (f) est de 0,005 ou plus.

**5.** Complexe de métal selon la revendication 3, dans lequel la différence d'énergie (S1 - T1) est supérieure à 0,28 (eV) et est de 1 (eV) ou moins et l'intensité d'oscillateur (f) est de 0,035 ou plus.

**6.** Complexe de métal selon l'une quelconque des revendications 2 à 4, dans lequel la différence d'énergie (S1 - T1 se situe dans une plage de 0,15 (eV) à 0,28 (eV) (les deux valeurs étant incluses) et l'intensité d'oscillateur (f) se situe dans une plage de 0,025 à 0,05 (les deux valeurs étant incluses).

**7.** Complexe de métal selon la revendication 3 ou 5, dans lequel la différence d'énergie (S1 - T1) est supérieure à 0,28 (eV) et est de 1,0 (eV) ou moins et l'intensité d'oscillateur (f) se situe dans une plage de 0,07 à 0,03 (les deux valeurs étant inclusses).

**8.** Complexe de métal selon la revendication 3 ou 5, dans lequel la différence d'énergie (S1 - T1) est supérieure à 0,28 (eV) et est de 1,0 (eV) ou moins et l'intensité d'oscillateur (f) se situe dans une plage de 0,09 à 0,03 (les deux valeurs étant incluses).

**9.** Complexe de métal selon la revendication 3 ou 5, dans lequel la différence d'énergie (S1 - T1) est supérieure à 0,28 (eV) et est de 1,0 (eV) ou moins et l'intensité d'oscillateur (f) se situe dans une plage de 0,10 à 0,25 (les deux valeurs étant incluses).

**10.** Complexe de métal selon l'une quelconque des revendications 2 à 9, qui est non ironique.

**11.** Complexe de métal selon l'une quelconque des revendications 2 à 10, comportant au moins un ligand de chélate multidenté contenant au moins un cycle aromatique.

**12.** Composition **caractérisée en ce qu'**elle comprend le complexe de métal selon l'une quelconque des revendications 1 à 11 et un matériau de transport de charge.

**13.** Composition selon la revendication 12, dans laquelle le matériau de transport de charge est un composé organique.

**14.** Composition selon la revendication 13, dans laquelle le composé organique est un composé organique à faible masse moléculaire.

**15.** Composition selon la revendication 13, dans laquelle le composé organique est un polymère.

**16.** Composition selon la revendication 15, dans laquelle le polymère est un polymère conjugué.

**17.** Composition selon la revendication 16, dans laquelle le polymère contient des motifs de répétition représentés par la formule générale (6) ci-dessous,

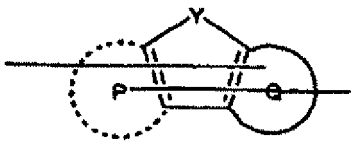

(6)

dans laquelle le cycle P et le cycle Q représentent chacun indépendamment un cycle aromatique ; toutefois, le cycle P peut ne pas être présent ; deux liaisons sont présentes sur le cycle P et/ou sur le cycle Q lorsque le cycle P est présent, et sont présentes sur le cycle à 5 chaînons ou le cycle à 6 chaînons contenant chacun Y et/ou sur le cycle Q lorsque le cycle P n'est pas présent ; un substituant peut être présent sur le cycle aromatique et/ou le cycle à 5 chaînons ou le cycle à 6 chaînons contenant Y ; Y représente -O-, -S-, -Se-, -B($R_{31}$)-, -N($R_{35}$)-, -C($R_1$) ($R_2$)-, -Si ($R_1$) ($R_2$)-, -P($R_3$)-, -P$R_4$(=O)-, -C($R_{51}$) ($R_{52}$)-C ($R_{53}$) ($R_{54}$)-, -O-C($R_{55}$) ($R_{56}$)-, -3-C($R_{57}$) ($R_{58}$)-, -N-C($R_{59}$) ($R_{60}$)-, -Si ($R_{61}$) ($R_{62}$) -C($R_{63}$) ($R_{64}$)-, -Si($R_{65}$) ($R_{66}$)-Si($R_{67}$)($R_{68}$)-, -C($R_{69}$)=C($R_{70}$)-, -N-C($R_{71}$)- ou -Si($R_{72}$)=C($R_{73}$)- ; $R_1$ à $R_4$, $R_{31}$, $R_{35}$ et $R_{51}$ à $R_{73}$ représentent chacun indépendamment un atome d'hydrogène, un groupe alkyle, un groupe alcoxy, un groupe alkylthio, un groupe aryle, un groupe aryloxy, un groupe arylthio, un groupe arylalkyle, un groupe arylalcoxy, un groupe arylalkylthio, un groupe arylalcényle, un groupe arylalcynyle, un groupe amino, un groupe amino substitué, un groupe silyle, un groupe silyle substitué, un groupe silyloxy, un groupe silyloxy substitué, un groupe hétérocyclique monovalent ou un atome d'halogène.

**18.** Polymère **caractérisé en ce qu'**il contient le complexe de métal selon l'une quelconque des revendications 1 à 11 comme structure partielle dans une molécule.

**19.** Polymère selon la revendication 18, qui est un polymère conjugué.

**20.** Composition **caractérisée en ce qu'**elle contient au moins un type de matériau choisi dans le groupe constitué de la composition ou du polymère selon l'une quelconque des revendications 12 à 19, un matériau de transport de trous, un matériau de transport d'électrons et un matériau d'émission de lumière.

**21.** Composition d'encre **caractérisée en ce qu'**elle contient le complexe de métal, la composition ou le polymère selon les revendications 1 à 20.

**22.** Composition d'encre selon la revendication 21, ayant une viscosité de 1 à 100 mPa·s à 25 °C.

**23.** Film fin d'émission de lumière **caractérisé en ce qu'**il contient le complexe de métal, la composition ou le polymère selon les revendications 1 à 20.

**24.** Dispositif photoélectrique **caractérisé en ce qu'**il contient le complexe de métal, la composition ou le polymère

selon les revendications 1 à 20.

**25.** Dispositif photoélectrique **caractérisé en ce qu'**il comprend une couche contenant la composition ou le polymère selon l'une quelconque des revendications 12 à 20 entre des électrodes constituées d'une anode et d'une cathode.

**26.** Dispositif photoélectrique selon la revendication 25, comprenant en outre une couche de transport de charge ou une couche de blocage de charge entre les électrodes constituées d'une anode et d'une cathode.

**27.** Dispositif photoélectrique selon l'une quelconque des revendications 24 à 26, dans lequel le dispositif photoélectrique est un dispositif d'émission de lumière.

**28.** Source de lumière plane **caractérisé en ce qu'**elle utilise le dispositif d'émission de lumière selon la revendication 27.

**29.** Dispositif d'affichage à segments **caractérisé en ce qu'**il utilise le dispositif d'émission de lumière selon la revendication 27.

**30.** Dispositif d'affichage à matrice à points **caractérisé en ce qu'**il utilise le dispositif d'émission de lumière selon la revendication 27.

**31.** Dispositif d'affichage crystal liquide **caractérisé en ce qu'**il utilise le dispositif d'émission de lumière selon la revendication 27 en tant que rétroéclairage.

**32.** Eclairage **caractérisé en ce qu'**il utilise le dispositif d'émission de lumière selon la revendication 27.

FIG. 1

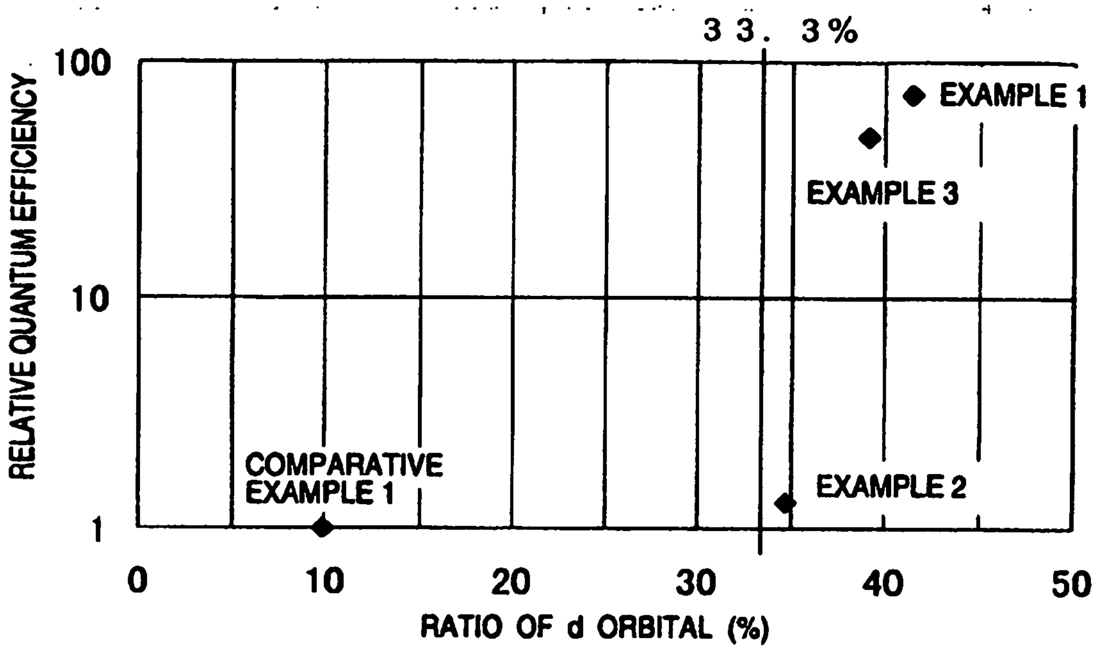
33.3%
100
10
1
RELATIVE QUANTUM EFFICIENCY
EXAMPLE 1
EXAMPLE 3
EXAMPLE 2
COMPARATIVE
EXAMPLE 1
0
10
20
30
40
50
RATIO OF d ORBITAL (%)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040183082 A
- JP 2003515897 A
- JP 2004244400 A
- JP 2004277377 A
- JP 2003231741 A
- JP 2004059899 A
- JP 204002654 A
- JP 2004292546 A
- US 5708130 A
- WO 9954385 A
- WO 0046321 A
- WO 02077060 A
- JP 57051781 A
- JP 59194393 A
- JP 63070257 A
- JP 63175860 A
- JP 2135359 A
- JP 2135361 A
- JP 2209988 A
- JP 3037992 A
- JP 3152184 A
- GB 2300196 A
- JP 3146296 B


### Non-patent literature cited in the description


- *APPLIED PHYSICS LETTERS,* 1999, vol. 75 (1), 4
- *JOURNAL OF PHYSICAL CHEMISTRY A,* 2002, vol. 106, 1634
- Organic Reactions. John Wiley & Sons, Inc, 1965, vol. 14, 270-490
- Organic Syntheses. John Wiley & Sons, Inc, 1988, 407-411
- Chem. Rev. 1995, vol. 95, 2457
- *J. Organomet. Chem.,* 1999, vol. 576, 147
- *J. Prakt. Chem.,* 1994, vol. 336, 247
- *Makromol. Chem., Macromol. Symp.,* 1987, vol. 12, 229
- *Inorg. Chem.,* 1984, vol. 23, 4249
- *Chem. Mater.,* 1999, vol. 11, 3709
- *Organometallics,* 1999, vol. 18, 1801
- *Inorg. Chem.,* 2002, vol. 41, 3055
- *J. Org. Chem.,* 1987, vol. 52, 73
- **SEISHI TOKITO ; TIHAYA ADACHI ; HIDEYUKI MURATA.** Organic EL Display. Ohmsha, Ltd, 2003, 107
- *Monthly Display,* 2003, vol. 9 (9), 26-30
- **SEISHI TOKITO ; TIHAYA ADACHI ; HIDEYUKI MURATA.** Organic EL Display. Ohmsha, Ltd, 111
- *Monthly Display,* 2002, vol. 9 (9), 47-51
- *Journal of the SID 11/1,* 2003, 161-166
- *Chem. Rev.,* 1989, vol. 89, 1359